# EUROPEAN PATENT SPECIFICATION

(11) **EP 1 984 334 B1**
(45) Date of publication and mention of the grant of the patent: **09.04.2014**
(21) Application number: 07756963.0
(22) Date of filing: 14.02.2007
(51) Int. Cl.: C07D 209/42, C07D 401/04, C07D 405/06, C07D 405/12, C07D 405/14, C07D 409/04

(54) **INDOLE-3-CARBOXYLIC ACID AMIDE, ESTER, THIOAMIDE AND THIOL ESTER COMPOUNDS BEARING ARYL OR HETEROARYL GROUPS HAVING SPHINGOSINE-1-PHOSPHATE (S1P) RECEPTOR ANTAGONIST BIOLOGICAL ACTIVITY**
INDOL-3-CARBONSÄUREAMID-, ESTER-, THIOAMID- UND THIOLESTERVERBINDUNGEN MIT ARYL- ODER HETEROARYLGRUPPEN MIT BIOLOGISCHER SPHINGOSIN-1-PHOSPHAT (S1P)-REZEPTORANTAGONISTENWIRKUNG
COMPOSES AMIDE D'ACIDE INDOLE-3-CARBOXYLIQUE, ESTER, THIOAMIDE ET ESTER DE THIOL PORTEURS DE GROUPES ARYL OU HETEROARYL PRESENTANT UNE ACTIVITE BIOLOGIQUE ANTAGONISTE SUR LE RECEPTEUR SPHINGOSINE-1-PHOSPHATE (S1P)

(30) Priority: 15.02.2006 US 774102 P; 11.01.2007 US 884470 P
(43) Date of publication of application: 29.10.2008
(73) Proprietor: ALLERGAN, INC., Irvine CA 92612 (US)
(72) Inventor: BEARD, Richard, L., Newport Beach, CA 92660 (US); DONELLO, John, E., Dana Point, CA 92629 (US); YUAN, Haiqing, Irvine, CA 92606 (US); LIU, Xiaoxia, Lake Forest, CA 92630 (US); DUONG, Tien, Rancho Santa Margarita, CA 92688 (US); COLON, Diana, F., Newport Beach, CA 92660 (US); HU, Yihui, Irvine, CA 92614 (US)
(74) Representative: HOFFMANN EITLE
(86) International application number: PCT/US2007/062106
(87) International publication number: WO 2007/095561

(56) References cited:
- WO-A-00/42045
- WO-A-01/98301
- WO-A-03/070691
- WO-A-2006/066879
- DE-A1- 19 753 522
- FR-A1- 2 121 394
- JP-A- 2007 126 454
- US-A- 5 994 378
- US-A1- 2003 220 319
- NAGASHIMA ET AL: "Fluorous 2-chloropyridinium salt (Mukaiyama condensation reagent) for amide formation reactions" TETRAHEDRON LETTERS, ELSEVIER, AMSTERDAM, NL, vol. 46, no. 38, 19 September 2005 (2005-09-19), pages 6585-6588, XP005034771 ISSN: 0040-4039
- DARYL R. SAUER ET AL.: "Microwave-assisted synthesis utilizing supported reagents: a rapid and efficient acylation procedure" ORGANIC LETTERS, vol. 5, no. 24, 2003, pages 4721-4724, XP002444821
- DOMSCHKE G ET AL: "N-SUBSTITUIERTE 1-BENZYL-2-METHYL-3-AMINOMETHYL-5-METHOXY- INDOLE UND VERWANDTE VERBINDUNGEN" CHEMISCHE BERICHTE, VERLAG CHEMIE GMBH. WEINHEIM, DE, vol. 93, 1960, pages 2097-2106, XP000961036 ISSN: 0009-2940
- KUTSCHY P ET AL: "Synthesis of some analogs of indole phytoalexins brassinin and methoxybras sinin B and their postional isomers" COLLECTION OF CZECHOSLOVAK CHEMICAL COMMUNICATIONS, INSTITUTE OF ORGANIC CHEMISTRY & BIOCHEMISTRY, PRAGUE, CZ, vol. 64, no. 2, February 1999 (1999-02), pages 348-362, XP002276545 ISSN: 0010-0765
- DI SANTO R ET AL: "N-(1-naphthylmethyl)-n-(1-alkyl-4-aryl-1h -pyrrol-3-ylmethyl)methylam ines related to naftifine. Synthesis and antifungal activity" MEDICINAL CHEMISTRY RESEARCH, BIRKHAEUSER, BOSTON, US, vol. 7, no. 2, 1997, pages 98-108, XP002958012 ISSN: 1054-2523
- DATABASE CHEMCATS CHEMICAL ASBTRACTS SERVICE, COLUMBUS, OHIOS, USA; XP002444828 & "Interchim Intermediates" 18 January 2005 (2005-01-18), INTERCHIM , 211 BIS AV JF KENNEDY, BP 1140, MONTLUCON, 03103, FRANCE
- JOGINDER S. BAJWA: "Chemoselective deprotection of benzyl esters in the presence of benzyl ethers, benzyloxymethyl ether and N-benzyl groups by catalytic transfer hydrogenation" TETRAHEDRON LETTERS, vol. 33, no. 17, 1992, pages 2299-2302, XP002444822
- JONATHAN CLAYDEN ET AL.: "Nucleophilic addition to electron-rich heteroaromatics: dearomatizing anionic cyclizations of pyrrolecarboxamides" ORGANIC LETTERS, vol. 6, no. 4, 2004, pages 609-611, XP002444823
- JOHN T. CARLOCK ET AL.: "3-diazo-4-oxo-3,4-dihydroquinoline. A novel synthon for indole-3-carboxamides" JOURNAL OF ORGANIC CHEMISTRY, vol. 42, no. 11, 1977, pages 1883-1885, XP002444824

## Description

### BACKGROUND OF THE INVENTION

### 1. Field of the Invention

The present invention relates to derivatives and/or analogues of sphingosine and pharmaceutical compositions, including such derivatives and/or analogues, which are useful as drugs for the treatment of fungal infections, allergic diseases, immune disorders, etc.

### 2. Summary of the Art

Sphingosine is a compound having the chemical structure shown in the general formula described below, in which Y¹ is hydrogen. It is known that various sphingolipids, having sphingosine as a constituent, are widely distributed in the living body including on the surface of cell membranes of cells in the nervous system.

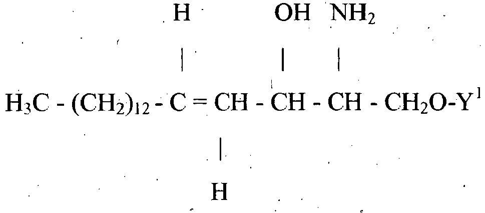

A sphingolipid is one of the lipids having important roles in the living body. A disease called lipidosis is caused by accumulation of a specified sphingolipid in the body. Sphingolipids present on cell membranes function to regulate cell growth; participate in the development and differentiation of cells; function in nerves; are involved in the infection and malignancy of cells; etc. Many of the physiological roles of sphingolipids remain to be solved. Recently the possibility that ceramide, a derivative of sphingosine, has an important role in the mechanism of cell signal transduction has been indicated, and studies about its effect on apoptosis and cell cycle have been reported.

Sphingosine-1-phosphate is an important cellular metabolite, derived from ceramide that is synthesized de novo or as part of the sphingomeyeline cycle (in animals cells). It has also been found in insects, yeasts and plants.

The enzyme, ceramidase, acts upon ceramides to release sphingosine, which is phosphorylated by spingosine kinase, a ubiquitous enzyme in the cytosol and endoplasmic reticulum, to form sphingosine-1-phosphate. The reverse reaction can occur also by the action of sphingosine phosphatases, and the enzymes act in concert to control the cellular concentrations of the metabolite, which concentrations are always low. In plasma, such concentration can reach 0.2 to 0.9 µM, and the metabolite is found in association with the lipoproteins, especially the HDL. It should also be noted that sphingosine-1-phosphate formation is an essential step in the catabolism of sphingoid bases.

Like its precursors, sphingosine-1-phosphate is a potent messenger molecule that perhaps uniquely operates both intra- and inter-cellularly, but with very different functions from ceramides and sphingosine. The balance between these various sphingolipid metabolites may be important for health. For example, within the cell, sphingosine-1-phosphate promotes cellular division (mitosis) as opposed to cell death (apoptosis), which it inhibits. Intracellularly, it also functions to regulate calcium mobilization and cell growth in response to a variety of extracellular stimuli. Current opinion appears to suggest that the balance between sphingosine-1-phosphate and ceramide and/or spingosine levels in cells is critical for their viability. In common with the lysophospholipids, especially lysophosphatidic acid, with which it has some structural similarities, sphingosine-1-phosphate exerts many of its extra-cellular effects through interaction with five specific G protein-coupled receptors on cell surfaces. These are important for the growth of new blood vessels, vascular maturation, cardiac development and immunity, and for directed cell movement. Thus, sphingosine ligands would be useful in the treatment of angiogenic disorders such as age-related macular degeneration (ARMD) and various cancers, etc.

Sphingosine-1 phosphate is stored in relatively high concentrations in human platelets, which lack the enzymes responsible for its catabolism, and it is released into the blood stream upon activation of physiological stimuli, such as growth factors, cytokines, and receptor agonists and antigens. It may also have a critical role in platelet aggregation and thrombosis and could aggravate cardiovascular disease. On the other hand the relatively high concentration of the metabolite in high-density lipoproteins (HDL) may have beneficial implications for atherogenesis. For example, there are recent suggestions that sphingosine-1-phosphate, together with other lysolipids such as sphingosylphosphorylcholine and lysosulfatide, are responsible for the beneficial clinical effects of HDL by stimulating the production of the potent antiatherogenic signaling molecule nitric oxide by the vascular endothelium. In addition, like lysophosphatidic acid, it is a marker for certain types of cancer, and there is evidence that its role in cell division or proliferation may have an influence on the development of cancers. These are currently topics that are attracting great interest amongst medical researchers, and the potential for therapeutic intervention in sphingosine-1-phosphate metabolism is under active investigation.

Fungi and plants have sphingolipids and the major sphingosine contained in these organisms has the formula described below. It is known that these lipids have important roles in the cell growth of fungi and plants, but details of the roles remain to be solved.

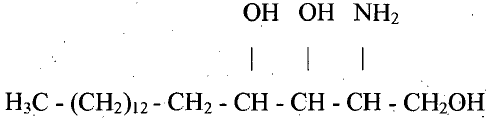

Recently it has been known that derivatives of sphingolipids and their related compounds exhibit a variety of biological activities through inhibition or stimulation of the metabolism pathways. These compounds include inhibitors of protein kinase C, inducers of apoptosis, immuno-suppressive compounds, antifungal compounds, and the like. Substances having these biological activities are expected to be useful compounds for various diseases.

Derivatives of sphingosine have been prepared in various patents. For example, see U.S. Patents 4,952,683; 5,110,987; 6,235,912 B1 and 6,239,297 B1.

Also, compounds which are similar to certain spingosine derivatives, but which are not reported as being ligands for the spingosine receptors are reported in various patents and published patent applications. See for example, U.S. Patents 5,294,722; 5,102,901; 5,403,851 and 5,580,878. U.S. Patent Application Publication No. U.S. 2003/0125371 A2. While certain of the compounds reported in the above patents are indoles, it does not appear that indole compounds have been reported as being ligands for sphingosine receptor or having activity as sphingosine agonists or antagonists.

WO 01/98301 discloses pyrazolopyridine compounds for use in the modulation of sphingosine-1-receptor.

### SUMMARY OF THE INTENTION

The present invention provides a compounds which are sphingosine-1-phosphate receptor agonists and/or antagonists, and pharmaceutical compositions comprising said compounds.

These compounds are represented by the formula I: 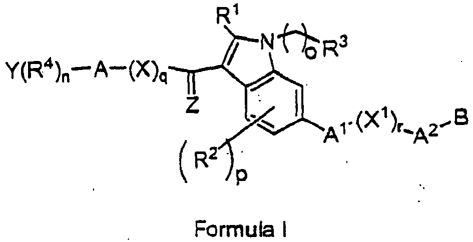 wherein:
R¹ R², R³ and R⁴ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, carbocyclic hydrocarbon groups having from 3 to 20 carbon atoms, heterocyclic groups having up to 20 carbon atoms and at least one of oxygen, nitrogen and/or sulfur in the ring, halo, C₁ to C₁₂ haloalkyl, hydroxyl, C₁ to C₁₂ alkoxy, C₃ to C₂₀ arylalkyloxy, C₁ to C₁₂ alkylcarbonyl, formyl, oxycarbonyl, carboxy, C₁ to C₁₂ alkyl carboxylate, C₁ to C₁₂ alkyl amide, aminocarbonyl, amino, cyano, diazo, nitro, thio, sulfoxyl, and sulfonyl groups;
X is NH;
X¹ is selected from the group consisting of NR⁵, O and S;
R⁵ is hydrogen, an alkyl group of 1 to 10 carbons, a cycloalkyl group of 5 to 10 carbons, phenyl or alkylphenyl wherein the alkyl group has 1-7 carbon atoms;
Y is a phenyl group or a pyridyl group, each of which may be bonded to A at any position;
Z is O;
n is 0 or an integer of from 1 to 5;
o is 0 or an integer of from 1 to 3;
p is 0 or an integer of from 1 to 3;
q is 1;
r is 0 or 1;
A is CH₂;
A¹ and A² are independently selected from the group consisting of (CH₂)ᵥ
wherein v is 0 or an integer of from 1 to 12, branched chain alkyl having 3 to 12 carbons, cycloalkyl having 3 to 12 carbons, alkenyl having 2 to 10 carbons and 1-3 double bonds and alkynyl having 2 to 10 carbons and 1 to 3 triple bonds;
B is selected from the group consisting of hydrogen, OR⁶, COOR⁷, NR⁸R⁹, CONR⁸R⁹, COR¹⁰, CH=NOR¹¹, CH=NNR¹²R¹³
wherein R⁶, R⁷, R¹⁰ and R¹¹ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, a carbocyclic hydrocarbon group having from 3 to 20 carbon atoms, a heterocyclic group having up to 20 carbon atoms and at least one of oxygen, nitrogen and/or sulfur in the ring, R⁸, R⁹, R¹² and R¹³ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, a carbocyclic hydrocarbon group having from 3 to 20 carbon atoms, a heterocyclic group having up to 20 carbon atoms and at least one of oxygen, nitrogen and/or sulfur in the ring, or R⁸ and R⁹ and/or R¹² and R¹³, together, can form a divalent carbon radical of 2 to 5 carbons to form a heterocyclic ring with nitrogen, wherein any of R⁶, R⁷, R⁸, R⁹, R¹⁰, R¹¹, R¹² or R¹³ may be substituted with one or more halogen, hydroxy, alkyloxy, cyano, nitro, mercapto or thiol radical; provided however, when v is 0, and r is 0, B is not hydrogen; or B is a carbocyclic hydrocarbon group having from 3 to 20 carbon atoms, or a heterocyclic group having up to 20 carbon atoms and at least one of oxygen, nitrogen and/or sulfur in the ring, and wherein when said B is a carbocyclic or heterocyclic group B may be bonded to A² at any position, or a pharmaceutically acceptable salt of said compound.

In the preferred embodiment of the present invention:

Preferably, n is 0 or an integer of 1 or 2 and R⁴ is fluoro.

Preferably, R¹ is i-propyl.

Preferably, R³ is selected from the group consisting of phenyl, which may be substituted with one or two flouro groups, and pyridyl.

Preferably, p is 0.

Preferably, A¹ and A² are absent, i.e. the index v is o for both A¹ and A².

Preferably, B is OR⁶ or COOR⁷.

Preferably, B is CR¹⁰=NOR¹¹R¹⁰ wherein R¹⁰ is H and R¹¹ is methyl or i-butyl or B is CONR⁸R⁹ wherein R⁸ and R⁹ are selected from the group consisting of H, methyl, ethyl and propyl, or R⁸ and R⁹, together with N, form a 5-member ring. Preferably, A¹ is absent, r is 0, A² is CH₂ and B is OR⁶, wherein R⁶ is H.

Specific Examples of the compounds of formula I include

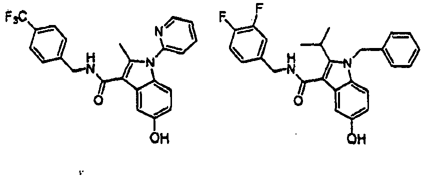

These compounds may be synthesized as illustrated by the synthesis scheme below:

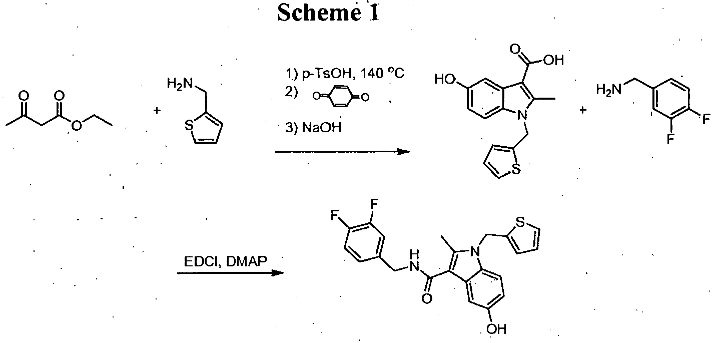

In general, a beta-ketoester (e.g. ethyl acetoacetate) is treated with an amine (e.g. 2-thiophenemethyl amine) in the presence of an organic acid (e.g. para-toluenesulfonic acid) and 1,4-benzoquinone to produce a 5-hydroxyindole-3-carboxylic acid (e.g. 5-hydroxy-2-methyl-1-(2-thiophenemethyl)indole-3-carboxylic acid) after hydrolysis of the ester with a strong base such as sodium hydroxide in a suitable solvent such as ethanol. The carboxylic acid is further reacted with an amine in the presence of *N*-(3-dimethylaminopropyl)-*N*'-ethylcarbodimide (EDC) to produce 5-hydroxyindole-3-carboxamide (e.g. 3,4-difluorophenylmethyl 5-hydroxy-2-methyl-1-(2-thiophenemethyl)indole-3-carboxamide). The carboxylic acid may also be treated with an alcohol or thiol in the presence of EDC to produce an ester and thiol ester derivatives, respectively. Specific Examples of the compounds of one of the preferred embodiments include

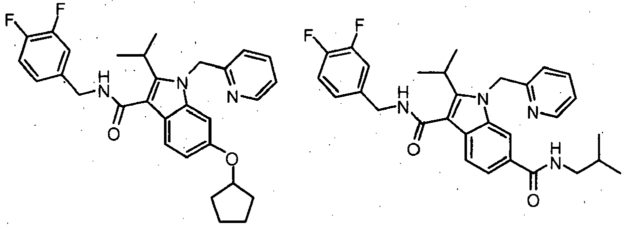

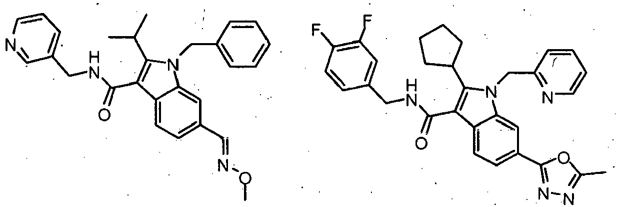

Some compounds within the scope of the invention may be prepared as depicted in Scheme 1. Thus, methyl 6-methoxyindole-2-carboxylate is treated with an electrophilic compound (e.g. benzyl bromide) in the presence of an weak base (e.g. potassium carbonate) to produce an N-alkylated indole (e.g. methyl 1-benzyl-6-methoxyindole-2-carboxylate). The 2-carboxylate group is converted to an alkyl group by a three-step process: Grignard reaction, elimination, and hydrogenation. The resulting 2-alkyl indole is carboxylated in the 3-position by treatment with dimethylformamide and phosphorus oxychloride followed by sodium hypochlorite oxidation of the resulting aldehyde. The carboxylic acid may be further functionalized by treatment with an amine in the presence of *N*-(3-dimethylaminopropyl)-*N*'-ethylcarbodimide (EDC) to produce a 6-methoxyindole-3-carboxamide derivative (e.g. 3,4-difluorophenylmethyl 6-methoxy-2-isopropyl-1-benzylindole-3-carboxamide). The carboxylic acid may also be treated with an alcohol or thiol in the presence of EDC to produce an ester and thiol ester derivatives, respectively. The 6-methoxy group may then be deprotected using boron tribromide and the resulting hydroxide subjected to alkylating (e.g. cyclopentyl iodide/potassium carbonate) or acylating (e.g. pivaloyl chloride/ pyridine) reagents to produce a large variety of 6-substituted indole homologs and derivatives within the scope of the invention.

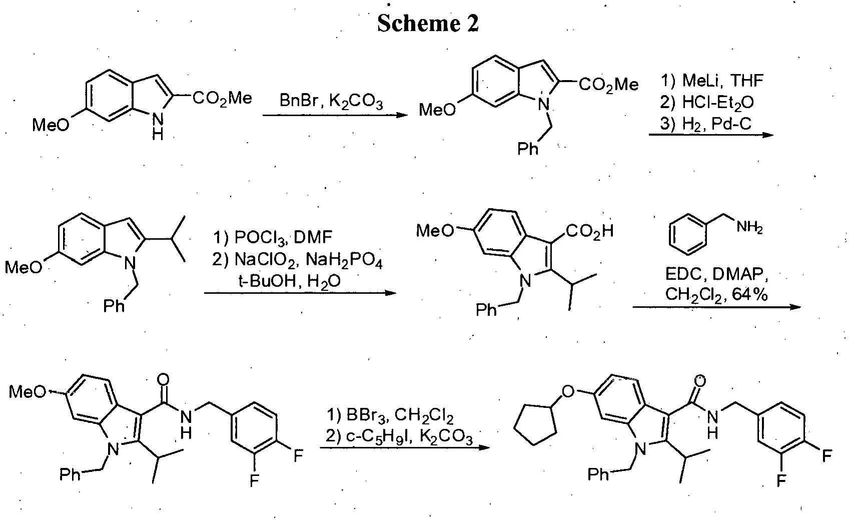

Many other compounds within the scope of the invention may be prepared as depicted in Scheme 2. Thus, ethyl 4-iodobenzoate may be nitrated in the 3-position with fuming nitric acid and the resulting nitro compound reduced under mild conditions (e.g. SnCl₂-H₂O) to produce ethyl 3-amino-4-iodobenzoate. This compound may be converted to the indole by treatment with a terminal alkyne (e.g. 3-methylbutyne) in the presence of a palladium catalyst and copper iodide followed by heating the aryl alkyne in the presence copper iodide. The resulting 2-alkyl indole may then be carbonylated in the 3-position by treatment with dimethylformamide and phosphorus oxychloride and N-alkylated as described above (benzyl bromide, potassium carbonate), followed by sodium hypochlorite oxidation to produce an N-alkylindole-3-carboxylic acid. The carboxylic acid may be further functionalized by treatment with an amine in the presence of EDC to 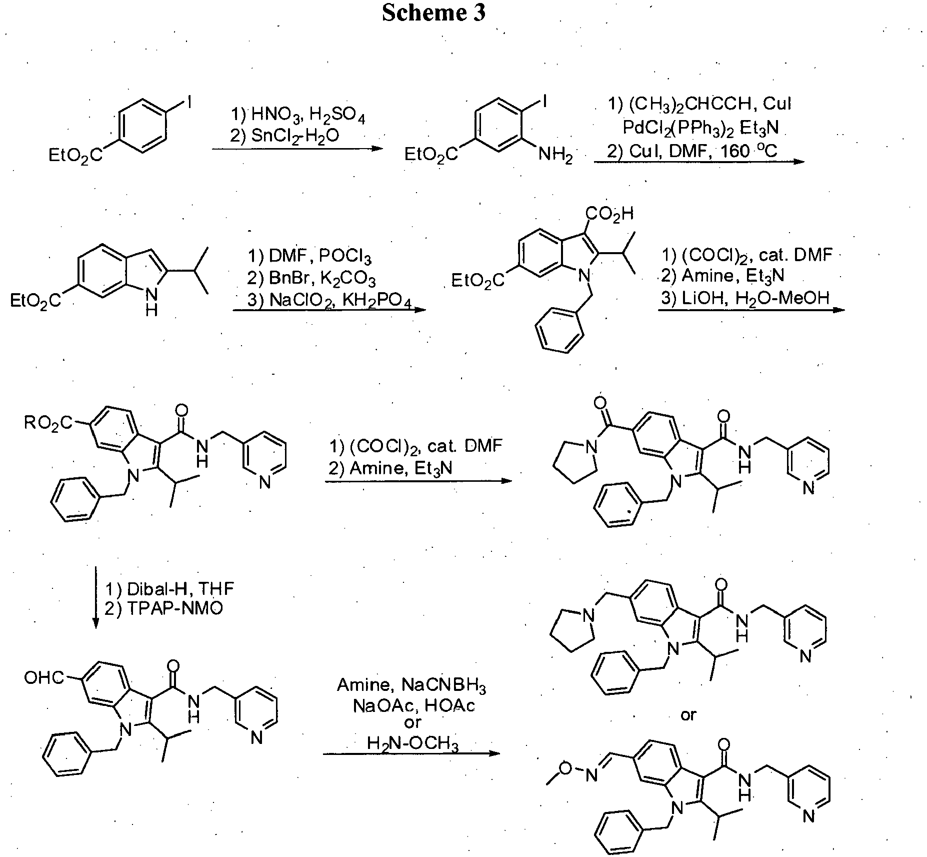 produce a 6-methoxyindole-3-carboxamide derivative (e.g. 3-pyridylmethyl 1-benzyl-6-carboethoxy-2-isopropylindole-3-carboxamide). The carboxylic acid may also be treated with an alcohol or thiol in the presence of EDC to produce an ester and thiol ester derivatives, respectively. The 6-carboethoxy group may be further functionalized to produce a large variety of 6-substituted indole homologs and derivatives within the scope of the invention. For example, the 6-carboethoxy group be hydrolyzed with strong base and the resulting carboxylic acid converted to the carboxylic acid chloride, which could be reacted with various alcohols or amines in the presence of base to produce ester or amide derivatives, respectively, such as 3-pyridylmethyl1-benzyl-2-isopropyl-6-(1-pyrrolidinylcarbamoyl)indole-3-carboxamide. Alternatively, the 6-carboethoxy group could be reduced to an alcohol and re-oxidized to an aldehyde intermediate, which may then be treated with an amine under reducing conditions to give amine derivatives such as 3-pyridylmethyl 1-benzyl-2-isopropyl-6-(1-pyrrolidinylmethyl)-indole-3-carboxamide. The aldehyde may also be treated with oxime or hydrazine compounds to produced oxime and hydrazone derivatives, respectively. Thus, many compounds within the scope of the invention may be produced by the general route depicted in Scheme 3.

The corresponding benzyl amino compounds may be prepared according to Scheme 4, below.

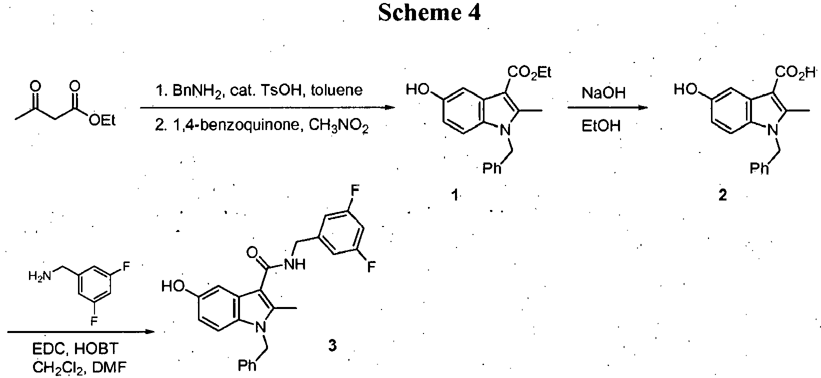

### DETAILED DESCRIPTION OF THE INVENTION

Unless otherwise indicated, the following terms as used throughout this specification have the following meanings:
"Me" refers to methyl.
"Et" refers to ethyl.
"tBu" refers to t-butyl.
"iPr" refers to i-propyl.
"Ph" refers to phenyl.
"Pharmaceutically acceptable salt" refers to those salts which retain the biological effectiveness and properties of the free bases and which are obtained by reaction with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like.
"Alkyl" refers to a straight-chain, branched or cyclic saturated aliphatic hydrocarbon. Preferably, the alkyl group has 1 to 12 carbons. More preferably, it is a lower alkyl of from 1 to 7 carbons, most preferably 1 to 4 carbons. Typical alkyl groups include methyl, ethyl, propyl, isopropyl, butyl, isobutyl, tertiary butyl, pentyl, hexyl and the like. The alkyl group may be optionally substituted with one or more substituents are selected from the group consisting of hydroxyl, cyano, alkoxy, =O, =S, NO₂, halogen, dimethyl amino and SH.
"Alkenyl" refers to a straight-chain, branched or cyclic unsaturated hydrocarbon group containing at least one carbon--carbon double bond. Preferably, the alkenyl group has 2 to 12 carbons. More preferably it is a lower alkenyl of from 2 to 7 carbons, most preferably 2 to 4 carbons. The alkenyl group may be optionally substituted with one or more substituents selected from the group consisting of hydroxyl, cyano, alkoxy, O, S, NO₂, halogen, dimethyl amino and SH.
"Alkynyl" refers to a straight-chain, branched or cyclic unsaturated hydrocarbon containing at least one carbon--carbon triple bond. Preferably, the alkynyl group has 2 to 12 carbons. More preferably it is a lower alkynyl of from 2 to 7 carbons, most preferably 2 to 4 carbons. The alkynyl group may be optionally substituted with one or more substituents selected from the group consisting of hydroxyl, cyano, alkoxy, O, S, NO₂, halogen, dimethyl amino and SH.
"Alkoxy" refers to an "O-alkyl" group.
"Aryl" refers to an aromatic group which has at least one ring having a conjugated pi electron system and includes carbocyclic aryl, heterocyclic aryl and biaryl groups. The aryl group may be optionally substituted with one or more substituents selected from the group consisting of halogen, trihalomethyl, hydroxyl, SH, OH, NO₂, amine, thioether, cyano, alkoxy, alkyl, and amino.
"Alkaryl" refers to an alkyl that is covalently joined to an aryl group. Preferably, the alkyl is a lower alkyl.
"Aryloxy" refers to an "O-aryl" group.
"Arylalkyloxy" refers to an "O-alkaryl" group.
"Carbocyclic aryl" refers to an aryl group wherein the ring atoms are carbon.
"Heterocyclic aryl" refers to an aryl group having from 1 to 3 heteroatoms as ring atoms, the remainder of the ring atoms being carbon. Heteroatoms include oxygen, sulfur, and nitrogen.
"Hydrocarbyl" refers to a hydrocarbon radical having only carbon and hydrogen atoms. Preferably, the hydrocarbyl radical has from 1 to 20 carbon atoms, more preferably from 1 to 12 carbon atoms and most preferably from 1 to 7 carbon atoms.
"Substituted hydrocarbyl" refers to a hydrocarbyl radical wherein one or more, but not all, of the hydrogen and/or the carbon atoms are replaced by a halogen, nitrogen, oxygen, sulfur or phosphorus atom or a radical including a halogen, nitrogen, oxygen, sulfur or phosphorus atom, e.g. fluoro, chloro, cyano, nitro, hydroxyl, phosphate, thiol, etc.
"Amide" refers to --C(O)--NH--R', wherein R' is alkyl, aryl, alkylaryl or hydrogen.
"Ester" refers to --C(O)--O--R', wherein R' is alkyl, aryl or alkylaryl.
"Thioamide" refers to --C(S)--NH--R', wherein R' is alkyl, aryl, alkylaryl or hydrogen.
"Thiol ester" refers to --C(O)--S--R', wherein R' is alkyl, aryl, alkylaryl or hydrogen.
"Amine" refers to a --N(R")R"' group, wherein R" and R"' are independently selected from the group consisting of alkyl, aryl, and alkylaryl.
"Thioether" refers to --S--R", wherein R" is alkyl, aryl, or alkylaryl.
"Sulfonyl" refers to --S(O)₂ --R"", where R"" is aryl, C(CN)=C-aryl, CH₂ CN, alkylaryl, sulfonamide, NH-alkyl, NH-alkylaryl, or NH-aryl.

Also, alternatively the substituent on the phenyl moiety, as shown below, is referred to as an o, m or p substituent or a 2, 3 or 4 substituent, respectively.

(Obviously, the 5 substituent is also a m substituent and the 6 substituent is an o substituent.)

Specific compounds of the invention are reported in Table I, below.

Compounds may be assessed for their ability to activate or block activation of the human S1P3 receptor in T24 cells stably expressing the human S1P3 receptor. In this assay ten thousand cells/well are plated into 384-well poly-D-lysine coated plates one day prior to use. The growth media for the S1P3 receptor expressing cell line is McCoy's 5A medium supplemented with 10% charcoal-treated fetal bovine serum (FBS), 1% antibiotic-antimycotic and 400 µg/ml geneticin. On the day of the experiment, the cells are washed twice with Hank's Balanced Salt Solution supplemented with 20 mM HEPES (HBSS/Hepes buffer). The cells are then dye loaded with 2 uM Fluo-4 diluted in the HBSS/Hepes buffer with 1.25 mM Probenecid and incubated at 37°C for 40 minutes. Extracellular dye is removed by washing the cell plates four times prior to placing the plates in the FLIPR (Fluorometric Imaging Plate Reader, Molecular Devices). Ligands are diluted in HBSS/Hepes buffer and prepared in 384-well microplates. The positive control, Sphingosine-1-Phosphate (S1P), is diluted in HBSS/Hepes buffer with 4 mg/ml fatty acid free bovine serum albumin. The FLIPR transfers 12.5 µl from the ligand microplate to the cell plate and takes fluorescent measurements for 75 seconds, taking readings every second, and then for 2.5 minutes, taking readings every 10 seconds. Drugs are tested over the concentration range of 0.61 nM to 10,000 nM. Data for Ca⁺² responses is obtained in arbitrary fluorescence units and not translated into Ca⁺² concentrations. IC₅₀ values are determined through a linear regression analysis using the Levenburg Marquardt algorithm.

**Table I S1P Receptor Antagonist Activity**

| **Compound Number** | Structure |
|---|---|
| **1** | 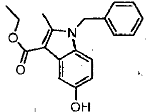 |
| **3** | 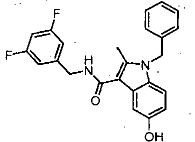 |
| **4** | 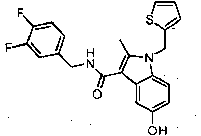 |
| **5** | 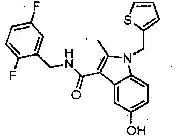 |
| **6** | 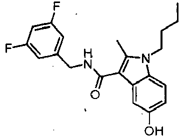 |
| **7** | 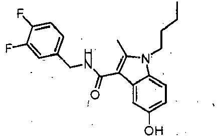 |
| **8** | 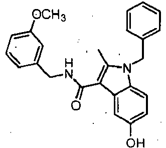 |
| **9** | 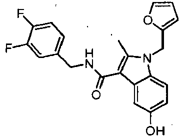 |
| **10** | 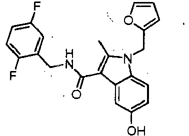 |
| **11** | 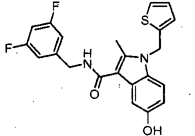 |
| **12** | 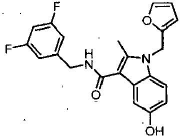 |
| **13** | 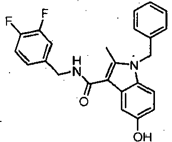 |
| **14** | 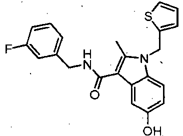 |
| **15** | 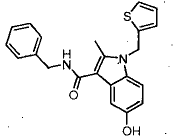 |
| **16** | 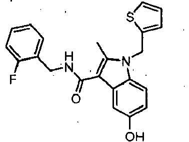 |
| **17** | 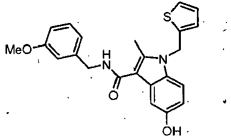 |
| **18** | 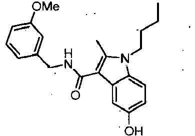 |
| **19** | 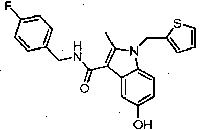 |
| **20** | 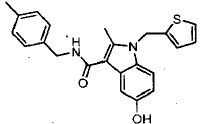 |
| **21** | 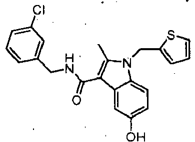 |
| **22** | 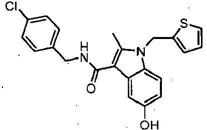 |
| **23** | 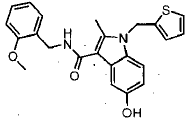 |
| **24** | 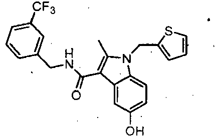 |
| **25** | 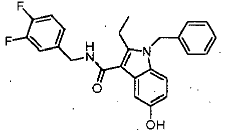 |
| **26** | 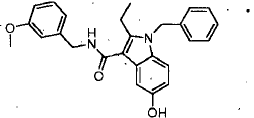 |
| **27** | 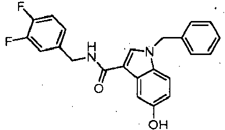 |
| **28** | 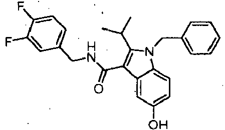 |
| **29** | 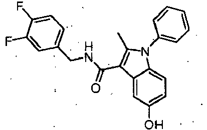 |
| **30** | 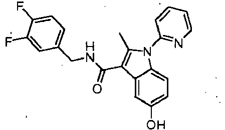 |
| **31** | 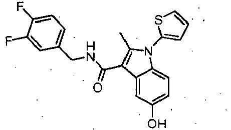 |
| **32** | 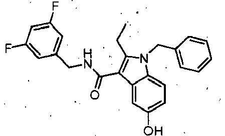 |
| **33** | 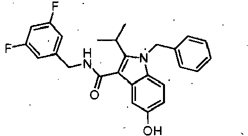 |
| **34** | 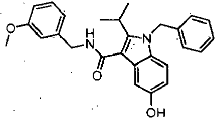 |
| **35** | 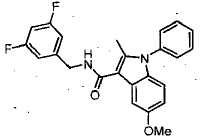 |
| **36** | 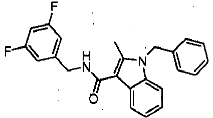 |
| **37** | 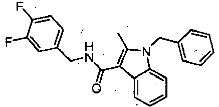 |
| **38** | 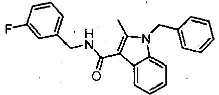 |
| **39** | 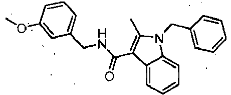 |
| **40** | 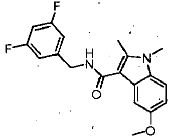 |
| **41** | 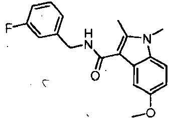 |
| **42** | 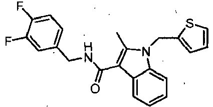 |
| **43** | 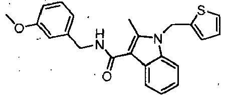 |
| **44** | 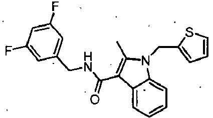 |
| **45** | 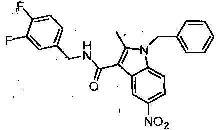 |
| **46** | 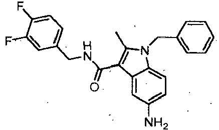 |
| **47** | 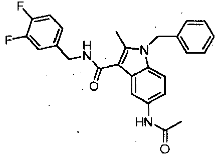 |
| **48** | 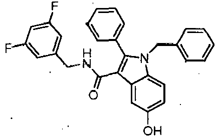 |
| **49** | 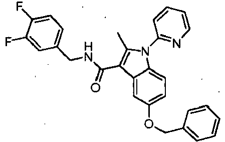 |

The preferred compounds of the invention are disclosed in Table 2 below:

**Table 2**

| **Compound Number** | Structure |
|---|---|
| **107** | 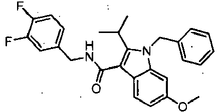 |
| **108** | 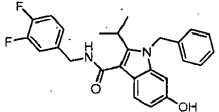 |
| **109** | 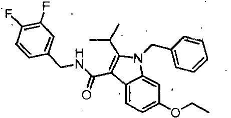 |
| **110** | 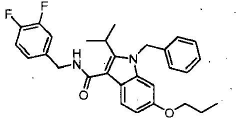 |
| **111** | 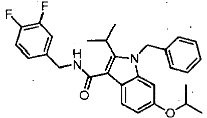 |
| **112** | 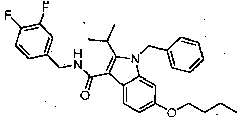 |
| **113** | 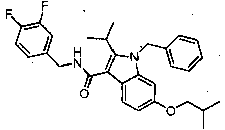 |
| **114** | 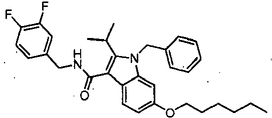 |
| **115** | 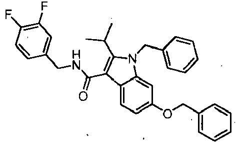 |
| **116** | 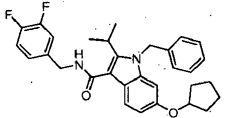 |
| **117** | 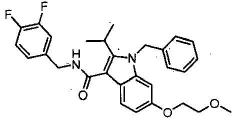 |
| **118** | 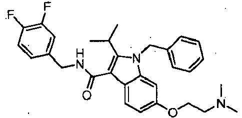 |
| **119** | 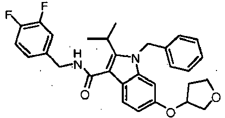 |
| **120** | 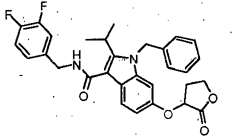 |
| **121** | 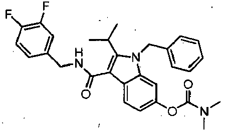 |
| **122** | 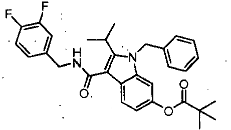 |
| **123** | 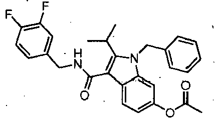 |
| **124** | 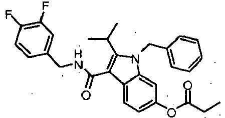 |
| **125** | 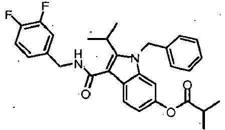 |
| **126** | 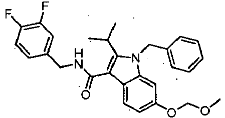 |
| **127** | 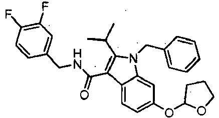 |

The compounds of Table 3 are prepared according to procedures analogous to the procedures of Schemes 1 through 4 and/or the Examples below.

**Table 3**

| **Compound Number** | Structure |
|---|---|
| | |
| | 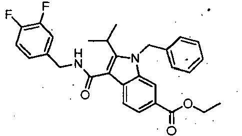 |
| | 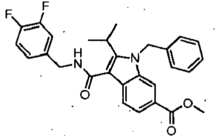 |
| | 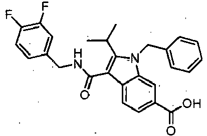 |
| | 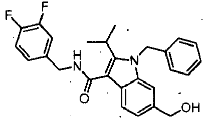 |
| | 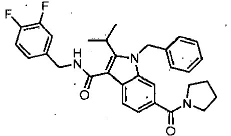 |
| | 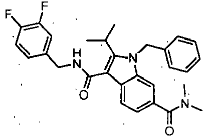 |
| | 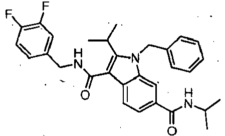 |
| | 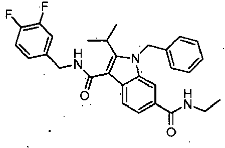 |
| | 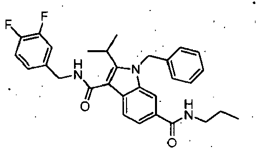 |
| | 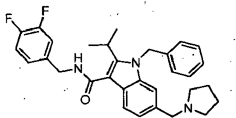 |
| | 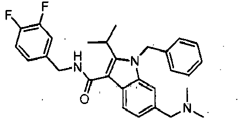 |
| | 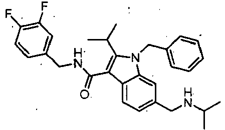 |
| | 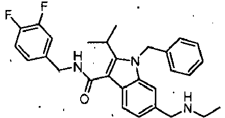 |
| | 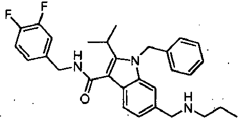 |
| | 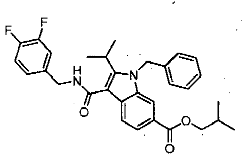 |
| | 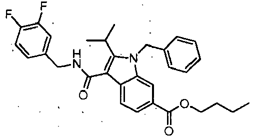 |
| | 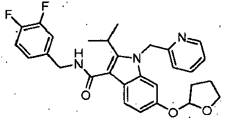 |
| | 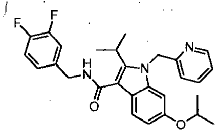 |
| | 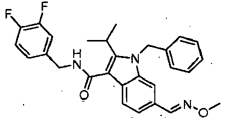 |
| | 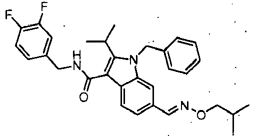 |
| | 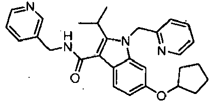 |
| | 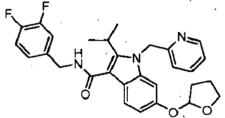 |
| | 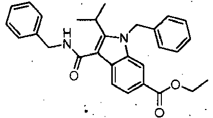 |
| | 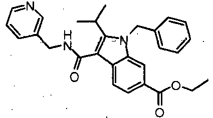 |
| | 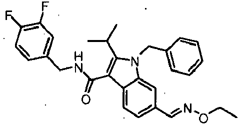 |
| | 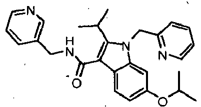 |
| | 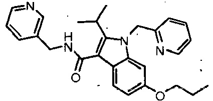 |
| | 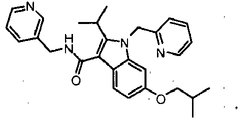 |
| | 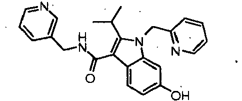 |
| | 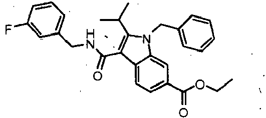 |
| | 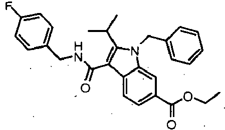 |
| | 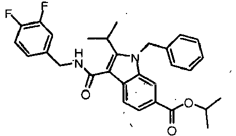 |
| | 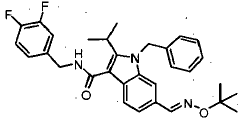 |
| | 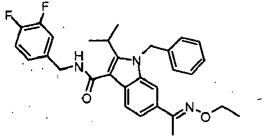 |
| | 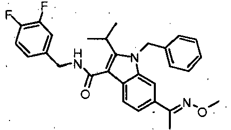 |
| | 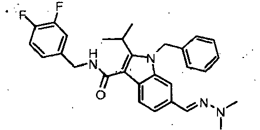 |
| | 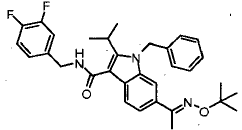 |
| | 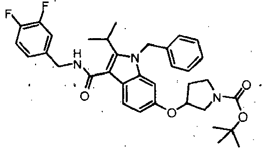 |
| | 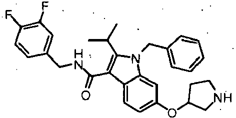 |
| | 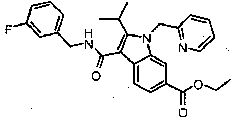 |
| | 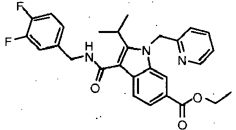 |
| | 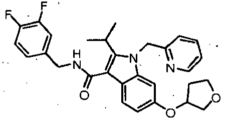 |
| | 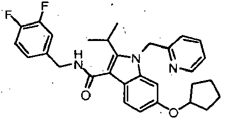 |
| | 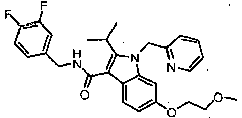 |
| | 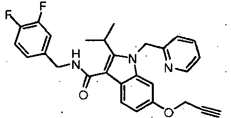 |
| | 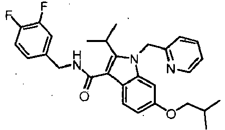 |
| | 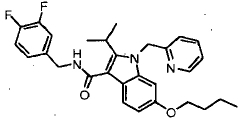 |
| | 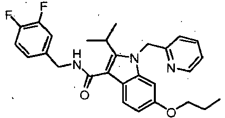 |
| | 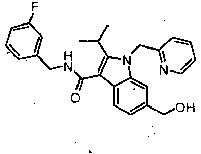 |
| | 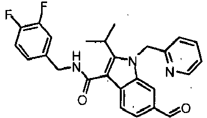 |
| | 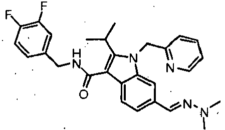 |
| | 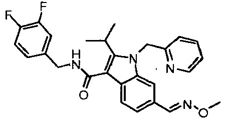 |

As a result of the activity of the compounds, i. e. as sphingosine ligands, the above compounds may be used in treating the following diseases and conditions for the following reasons.
**Glaucoma**
   S1P3 subtypes are expressed in primary human trabecular meshwork cells and S1P decreases outflow facility >30% in perfused porcine eyes (See IOVS 45, 2263; 2004) by altering paracellular permeability.
**Dry Eye/Immunology**
   Induces lymphocyte sequestration without affecting T cell proliferation.
**Angiogenesis disorders**
   S1P3 receptor subtype is expressed in vascular endothelial cells and siRNA knockdown of S1P1 and S1P3 inhibits angiogenesis. S1P also promotes vascular endothelial cell migration and promotes barrier assembly and integrity.
**Cardiovascular (S1P3)**
   S1P3 "knock out" mice lack S1P induced pulmonary edema.
**Additional Clinical Potential of S1P Receptors**
**Selective Agonist and Antagonist Ligands of the Invention**

### S1P3 blockage for protecting epithelial integrity in pulmonary disease like acute respiratory distress syndrome

Acute respiratory distress syndrome (ARDS) is a serious reaction to various forms of injuries to the lung. This is the most important disorder resulting in increased permeability pulmonary edema. The annual incidence of ARDS is between 1.5 to 13.5 people per 100,000 in the general population. Mechanical ventilation, sepsis, pneumonia, shock, aspiration, trauma (especially pulmonary contusion), major surgery, massive transfusions, smoke inhalation, drug reaction or overdose, fat emboli and reperfusion pulmonary edema after lung transplantation or pulmonary embolectomy may all trigger ARDS. Pneumonia and sepsis are the most common triggers, and pneumonia is present in up to 60% of patients. Pneumonia and sepsis may be either causes or complications of ARDS. It is hard to find a good method to treat ARDS in clinic. The most reliable treatment is ventilation with positive end-exporatory pressure (PEEP). Pulmonary pathologies including adult respiratory distress syndrome are characterized by disruption of pulmonary integrity and edema compromising respiratory function. Sphingosine 1-phosphate (S1P) is a lipid mediator synthesized and/or stored in mast cells, platelets, and epithelial cells, with production up-regulated by the proinflammatory cytokines IL-1 and TNF. S1P administration via the airways but not via the vasculature induces lung leakage. Using receptor-null mice, results show that S1P, acting on S1P₃ receptor expressed on both type I and type II alveolar epithelial cells but not vascular endothelium, induces pulmonary edema by acute tight junction opening. WT but not S1P₃-null mice showed disruption of pulmonary epithelial tight junctions and the appearance ofparacellular gaps between epithelial cells by electron microscopy within 1 h of airways exposure to S1P. S1P shows synergistic activity with the proinflammatory cytokine TNF, showing both pulmonary edema and mortality at subthreshold S1P doses. Specifically, preexposure of mice to subthreshold doses of TNF, which alone induced no lung edema, exacerbated S1P-induced edema and impaired survival. S1P, acting through S1P₃, regulates epithelial integrity and acts additively with TNF in compromising respiratory barrier function. Because S1P₃-null mice are resistant to S1P-induced pulmonary leakage, either alone or in the presence of TNF, S1P₃ antagonism may be useful in protecting epithelial integrity in pulmonary disease.

Based on the data that show S1P, acting through the S1P3 receptor expressed on pulmonary epithelium, is an acute regulator of epithelial integrity by disrupting tight junctions. SIP3 blockers are believed to be useful for treating ARDS.

### Regulation of S1P signaling EDG receptor pathway to improve the condition of congestive cardiac failure

Congestive heart failure (CHF), also called congestive cardiac failure (CCF) or just heart failure, is a condition that can result from any structural or functional cardiac disorder that impairs the ability of the heart to fill with or pump a sufficient amount of blood throughout the body. The most common cause of heart failure is ischemic heart disease (myocardial infarction of coronary artery disease). Left heart failure will increase hypertension in pulmonary system, and fail to remove the fluid from the lung circulation to lead pulmonar edema.

There is a vicious circle in left heart failure and lung edema. Heart ischemia and infarction reduce myocarial contraction to induce heart failure, pulmonary hepertension, and pulmonary edema. Pulmonary edema inrease barrier of oxygen from lung to blood of lung circulation, then Po2 in artery will sharply decrease. The suppliment of oxygen to heart will short to requirement for keeping contraction force, then enforce heart failure.

The most common cause of CHF in United States is ischemic heart disease. The treatment of CHF in clinic includes improving heart function, reducing heart preload and afterload. Research demonstrated that S1P signaling Edg receptor pathway may be involved in regulation of cardiovascular systems and pulmonary leakage. S1P acutely protect the heart against ischemia/reperfusion injury, decrease the region of infarction of myocardial ischemia, and keep coronary artery in dilation. S1P also can enhance pulmonary endothelial cell barrier to reduce lung edema that was induced by left heart failure. Antagonist of S1P3 receptor can block contraction of vascular smooth muscle, and decrease heart afterload. Blockage of S1P3 receptor also can prevent one of S1P toxicity, bradycardia.

### Treatment of asthma and chronic obstructive pulmonary disease

Asthma is a chronic disease of the respiratory system in which the airway occasionally constricts, becomes inflamed, and is lined with excessive amounts of mucus, often in response to one or more triggers. In response to exposure to these triggers, the bronchi contract into spasm (an "asthma attack"). Inflammation soon follows, leading to a further narrowing of the airways and excessive mucus production, which leads to coughing and other breathing difficulties.

Chronic obstructive pulmonary disease (COPD) belongs next to bronchial asthma to the most important diseases of the respiratory tract. COPD is a major global health problem, and is predicted to become the third most common cause of death by 2020. COPD is a disease characterized by progressive airflow obstruction of the peripheral airways, associated with lung inflammation, emphysema and mucus hypersecretion.

The major problems in asthme and COPD are bronchi or small bronchi constraction, and inflamation. Recently, many science papers reported S1P involed in constrction of human airway smooth muscle cells. S1P stimulates constrction of human airway smooth muscle cells by S 1 P3 receptors. Recent work has revealed that levels of S1P are elevated in the airways of asthmatics and not in healthy individuals after segmental allergan challenge. Research also demonstrates that S1P signaling pathway contributes to cholinergic constriction of murine peripherial airways.

Thus, S1P3 receptor is a potential new therapeutic target for asthma and COPD. S1P3 antagonist would block the stimulation of S1P to human airway smooth muscle cell S1P3 receptor preventing bronchial constraction.

### Use of S1P3 receptor selective antagonist in controlling human hypertension

Hypertension, commonly referred to as "high blood pressure", is a medical condition where the blood pressure is chronically elevated. Persistent hypertension is one of the risk factors for strokes, heart attacks, heart failure and arterial aneurysm, and is a leading cause of chronic renal failure. Currently, it is estimated that 58 million adults in the United States have hypertension or are taking antihypertensive medications. In addition to definitive hypertension, an additional 45 million adults in the United States have pre-hypertension.

Recently, it has been found that S1P, a bioactive lipid mediator, involved in cardiovascular system, and cholesterol metabolism. Evidences link S1P₃ receptor activity with acute toxicity and cardiovascular regulation: compound potency on S1P₃ correlated with toxicity and bradycardia; the shift in potency of phosphorylated-FTY720 for inducing lymphopenia versus bradycardia and hypertension was consistent with affinity for S1P₁ relative to S1P₃; and toxicity, bradycardia, and hypertension were absent in S1P₃^{-/-} mice. Blood pressure effects of agonists in anesthetized rats were complex, whereas hypertension was the predominant effect in conscious rats and mice. Immunolocalization of S1P₃ in rodent heart revealed abundant expression on myocytes and perivascular smooth muscle cells consistent with regulation of bradycardia and hypertension, whereas S1P₁ expression was restricted to the vascular endothelium. In conclusion, hypertension is clearly associated with S1P3 activation and its expression patterns in cardiovascular tissue. S1P and S 1 P3 receptor is believe to play an important role in the regulation of blood pressure.

### Heart rate regulation of S1P3 signaling pathway may be used for controlling techycardia, one of most common cardiac arrithymias

Tachycardia refers to a rapid beating of the heart. By convention the term refers to heart rates greater than 100 beats per minute in the adult patient. Tachycardia may be a perfectly normal physiological response to stress. However, depending on the mechanism of the tachycardia and the health status of the patient, tachycardia may be harmful, and require medical treatment. In extreme cases, tachycardia can be life threatening.

Tachycardia can be harmful in two ways: First, when the heart beats too rapidly, it may perform inefficiently. Second, the faster the heart beats, the more oxygen and nutrients the heart requires. This can be especially problematic for patients suffering from ischemic heart disease.

Sphingosine 1-phosphate (S1P) influences heart rate, coronary artery caliber, endothelial integrity, and lymphocyte recirculation through five related high affinity G-protein-coupled receptors. Circumstantial evidence largely from cultured atrial myocytes and using suramin inhibition as a measureof S1P₃ function has postulated a role for S1P₃ in the activation of an *I*_{KAch} channel-inducing bradycardia. The demonstration *in vivo* that a non-selective S1P receptor agonist active on S1P₃ induces bradycardia in wild-type mice that is abolished in S1P₃-/- mice provides further support for the role of S1P₃ in the heart. Both S1P₁ and S1P₃ are expressed on cardiac endothelium and perhaps myocardium, yet deletion of S1P₃ alone abolishes the bradycardia induced by non-selective S1P receptor agonists, and an S1P₁-selective agonist does not induce bradycardia. The sphingolipid drug FTY720 displays structural similarity to S1P and efficacy as an immunosuppressant in models of autoimmune disease and in solid organ transplantation. While FTY720 is well-tolerated in humans, it produces a transient reduction of heart rate (HR). As S1P activates the cardiac G protein-gated potassium channel IKACh, FTY720-induced HR reduction reflects IKACh activation. In wild-type myocytes, the active phosphate metabolite of FTY720 (FTY720-P) induced single channel activity with conductance, open time, GTP sensitivity and rectification identical to that of IKACh. In whole-cell recordings, FTY720-P evoked an inwardly rectifying potassium current in ~90% of myocytes responding to acetylcholine. Comparable channel activity was never observed in myocytes from IKACh-deficient mice. In wild-type mice, acute FTY720 administration produced a dose-dependent, robust HR reduction. In contrast, the HR reduction induced by FTY720 in IKACh-deficient mice was blunted. Research concludes that the effect of acute FTY720 administration on HR is mediated primarily by IKACh activation.

### Clinical potential of S1P signaling EDG receptors pathway in protecting myocardial ischemia and reperfusion injury

myocardial infarction, commonly known as a heart attack, is a disease state that occurs when the blood supply to a part of the heart is interrupted. The resulting oxygen shortage causes damage and potential death of heart tissue. It is a medical emergency, and the leading cause of death for both men and women all over the world. The main therapeutic goals in patients with acute myocardial infarction are to minimize myocardial damage, inprove cardiac repair, and reduce myocardial remodeling. State-of-the-art therapy is rapid reperfusion of the infarcted myocardium through revascularization of the occluded vessel. However, the benefit of reperfusion is compromised by the endothelial injury and inflammation that follow reinstitution of blood flow, leading to additional myocardial damage, a process termed "ischemia/referfusion injury." Despite all efforts to prevent the sequelae of referfusion injury in patients, there are currently no clinical strategies availble to effectively protect cardiac tissue from the inflammatory damage inherent reperfusion.

S1P is a bioactive phospholipid that is primarily stored in platelets. Platelets play a key role in the response to acute vascular injury. S1P potently affects the development and function of the heart. Mutation in a zebrafish S1P receptor called Miles Apart (which is most similar to the S1P₂ receptor of mammals) results in a myocardial precursor cell migration defect and formation of the cardia bifida condition, wherein the two primitive heart tube structures fail to coalesce and form a single mature heart structure. Studies in adult cardiac cells showed that S1P regulates the calcium metabolism and ionic currents in cells of the sinoatrial node, which controls heart rate. In addition, S1P was shown to prevent death of cardiac myocytes on ischemia/reperfusion injury. Cardiomyocytes express S1P₁₋₃ receptors, suggesting that signaling pathways stimulated by these receptors may contribute to intrinsic myocardial function. In an animal model of ischemia/reperfusion injury of the heart, S1P was found to be cardioprotective. In a mouse model of myocardial ischemia/reperfusion, S1P dramatically attenuated infarction size by approximately 20% and 40%, respectively. The underlying mechanism was an inhibition of inflammatory neutrophil recruitment and cardiomyocyte apoptosis in the infarcted area. S1P potently suppressed leukocyte adhesion to activated endothelium under flow and protected rat neonatal cardiomyocytes against apoptosis.

The invention is further illustrated by the following examples which are illustrative of a specific mode of practicing the invention and are not intended as limiting the scope of the claims.

Unless otherwise indicated, the following Chemical Abbreviations are used in the examples:
NaOH: sodium hydroxide
EtOH: ethanol
HCl: hydrogen chloride
EtOAc: ethyl acetate
Na₂SO₄: sodium sulfate
MeOH: methanol
Pd-C: palladium on activated carbon
Et₂O: diethyl ether
EDC: *N*-(3-Dimethylaminopropyl)-*N'*-ethylcarbodiimide hydrochloride
HOBT: 1-hydroxybenzotriazole
CH₂Cl₂: methylene chloride
DMF: *N,N*-dimethylformamide
DCC: *N,N'*-dicyclohexylcarbodiimide
DMSO: dimethylsulfoxide
Benzylamine, benzyl bromide, n-butylamine, 3-chlorobenzylamine, 4-chlorobenzylamine, furfuryl amine, 2,5-difluorobenzylamine, 3,4-difluorobenzylamine, 3,5-difluorobenzylamine, iodobenzene, 2-iodopyridine, 2-iodothiophene, ethyl acetoacetate, ethyl benzoylacetate, ethyl 5-hydroxy-2-methylindole-3-carboxylate, ethyl isobutyrylacetate, ethyl 3-oxovelarate, 2-fluorobenzylamine, 3-fluorobenzylamine, 4-fluorobenzylamine, 2-methoxybenzylamine, 3-methoxybenzylamine, 4-methylbenzylamine, 2-thiophenemethylamine and 3-(trifluoromethyl)benzylamine were purchased from Aldrich Chemical Company.
5-Benzyloxyindole-3-carboxaldehyde was purchased from Sigma Chemical Company.
2-Methyl-5-nitro-1H-indole-3carboxaldehyde was purchased from Fisher Scientific Company.

### Example 1

### Ethyl 1-Benzyl-5-hydroxy-2-methyl-1H-indole-3-carboxylate (Compound 1).

**General Procedure 1.** To a solution of ethyl acetoacetate (1.3 ml, 10 mmol) and benzylamine (1.2 ml, 10.5 mmol) in toluene (10 ml) was added p-toluenesulfonic acid monohydrate (95 mg, 0.5 mmol). The mixture was heated at 140 °C to reflux for 4 h, cooled to 0 °C and filtered. The filtrate was concentrated under reduced pressure to give a yellow oil (2.6 g). To a solution of 1,4-benzoquinone (1.49 g, 13.8 mmol) in nitromethane (5 ml) was added a solution of the above yellow oil in nitromethane (3.5 ml) slowly. The resulting mixture was stirred at room temperature for 18 h and was cooled to 0 °C and filtered. The solid was washed with cold nitromethane to yield ethyl 1-benzyl-5-hydroxy-2-methyl-1H-indole-3-carboxylate (**Compound 1**) as a beige solid.
¹H-NMR (CHLOROFORM-*d*) δ 1.45 (t, *J* = 7.0 Hz, 3 H), 2.70 (s, 3 H), 4.40 (q, *J* = 7.2 Hz, 2 H), 5.09 (s, 1 H), 5.31 (s, 2 H), 6.75 (dd, *J* = 8.6, 2.5 Hz, 1 H), 6.92-7.01 (m, 2 H), 7.08 (d, *J* = 8.8 Hz, 1 H), 7.23-7.32 (m, 3 H), 7.65 (d, *J* = 2.6 Hz, 1 H).

### Example 2

### 1-Benzyl-5-hydroxy-2-methyl-1H-indole-3-carboxylic Acid (Compound 2).

**General Procedure 2** A solution of ethyl 1-benzyl-5-hydroxy-2-methyl-1H-indole-3-carboxylate (**Compound 1**, 873 mg, 2.83 mmol) and NaOH (2.2 g, 56 mmol) in EtOH (10 ml) and H₂O (10 ml) was heated to 90 °C for 16 h. The reaction was quenched with 6M HCl (10 ml), extracted with EtOAc, washed with brine, dried over Na₂SO₄, and concentrated under reduced pressure. The residue was purified by flash column chromatography on silica gel (30% EtOAc-hexanes to 20% MeOH-EtOAc) to yield 1-benzyl-5-hydroxy-2-methyl-1H-indole-3-carboxylic acid (**Compound 2**) as a reddish brown solid.
¹H-NMR (METHANOL-*d*₄) δ 2.67 (s, 3 H), 5.41 (s, 2 H), 6.68 (dd, *J* = 8.8, 2.3 Hz, 1 H), 6.96-7.03 (m, 2 H), 7.15 (d, *J* = 8.8 Hz, 1 H), 7.20-7.32 (m, 3 H), 7.55 (d, *J* = 2.1 Hz, 1 H).

### Example 3.

**1-Benzyl-5-hydroxy-2-methyl-1H-indole-3-carboxylic Acid, 3,5-Difluorobenzylamide (Compound 3). General Procedure 3.** To a solution of 1-benzyl-5-hydroxy-2-methyl-1H-indole-3-carboxylic acid (**Compound 2, 205** mg, 0.73 mmol) in CH₂Cl₂ (5 ml) and DMF (3 ml) was added EDC (211 mg, 1.1 mmol), HOBT (149 mg, 1.1 mmol) and 3,5-difluorobenzylamine (260 µl, 2.2 mmol). The mixture was stirred at room temperature for 16 h, diluted with EtOAc, and washed with 1M HCl, and brine, and dried over Na₂SO₄, and concentrated under reduced pressure. The residue was purified by flash column chromatography on silica gel (30% to 50% EtOAc-hexanes) to yield I-benzyl-5-hydroxy-2-methyl-1H-indole-3-carboxylic acid, 3,5-difluorobenzylamide (**Compound 3**) as a beige solid.
¹H-NMR (METHANOL-*d*₄) δ 2.57 (s, 3 H), 4.61 (s, 2 H), 5.40 (s, 2 H), 6.70 (dd, *J* = 8.8, 2.3 Hz, 1 H), 6.77-6.88 (m, 1 H), 6.97-7.07 (m, 4 H), 7.14-7.19 (m, 1 H), 7.20-7.32 (m, 4 H).

**The following compounds were prepared using General Procedures 1, 2 and** 3 **and the appropriate amines and beta-ketoester starting materials, which are available from Aldrich Chemical Company or prepared as described below:**

### Example 4

### 5-Hydroxy-2-methyl-1-thiophen-2-ylmethyl-1H-indole-3-carboxylic Acid, 3, 4-Difluorobenzylamide (Compound 4).

¹H-NMR (ACETONE-*d*₆) δ 2.73 (s, 3 H), 4.64 (d, *J* = 6.1 Hz, 2 H), 5.59 (s, 2 H), 6.77 (dd, *J* = 8.5, 2.0 Hz, 1 H), 6.93-7.01 (m, 2 H), 7.25-7.40 (m, 6 H), 7.88 (br s, 1 H).

### Example 5

### 5-Hydroxy-2-methyl-1-thiophen-2-ylmethyl-1H-indole-3-carboxylic Acid, 2, 5-Difluorobenzylamide (Compound 5).

¹H-NMR (ACETONE-*d*₆) δ 2.74 (s, 3 H), 4.68 (d, *J* = 5.9 Hz, 2 H), 5.59 (s, 2 H), 6.77 (dd, *J* = 8.8, 2.4 Hz, 1 H), 6.93-7.33 (m, 6 H), 7.38 (d, *J* = 15.6 Hz, 2 H), 7.9 (br s, 1 H).

### Example 6

### 1-Butyl-5-hydroxy-2-methyl-1H-indole-3-carboxylic Acid, 3, 5-Difluoro-benzylamide (Compound 6).

¹H-NMR (CHLOROFORM-*d*) δ 0.95 (t, *J* = 7.5 Hz, 3 H), 1.38 (m, 2 H), 1.70 (m, 2 H), 2.69 (s, 3 H), 4.03 (t, *J* = 7.5 Hz, 2 H), 4.59 (d, *J* = 6.1 Hz, 2 H), 5.81 (s, 1 H), 6.23 (br t, I H), 6.66 (m, 1 H), 6.80 (dd, *J* = 2.2, 8.8 Hz, 1 H), 6.86 (br d, 2 H), 7.15 (br d, 2 H).

### Example 7

### 1-Butyl-5-hydroxy-2-methyl-1H-indole-3-carboxylic Acid, 3, 4-Difluoro-benzylamide (Compound 7).

¹H-NMR (CHLOROFORM-*d*) δ 0.95 (t, *J* = 7.5 Hz, 3 H), 1.36 (m, 2 H), 1.70 (m, 2 H), 2.69 (s, 3 H), 4.03 (t, *J* = 7.5 Hz, 2 H), 4.58 (d, *J* = 6.1 Hz, 2 H), 5.75 (s, 1 H), 6.20 (br t, 1 H), 6.76 (dd, *J* = 2.6, 8.8 Hz, 1 H), 7.05-7.16 (m, 5 H).

### Example 8

### 1-Benzyl-5-hydroxy-2-methyl-1H-indole-3-carboxylic Acid, 3-Methoxybenzylamide (Compound 8).

¹H-NMR (CHLOROFORM-*d*) δ 2.67 (s, 3 H), 3.81 (s, 3 H), 4.67 (d, *J* = 5.6 Hz, 2 H), 5.30 (s, 2 H), 6.17 (t, *J* = 5.6 Hz, 1 H), 6.73 (dd, *J* = 8.8, 2.3 Hz, 1 H), 6.83 (dd, *J* = 7.8, 2.2 Hz, 1 H), 6.94-7.02 (m, 4 H), 7.09 (d, *J* = 9.1 Hz, 1 H), 7.22 (d, *J* = 2.3 Hz, 1 H), 7.23-7.33 (m, 4 H).

### Example 9

### 1-Furan-2-ylmethyl-5-hydroxy-2-methyl-1H-indole-3-carboxylic Acid, 3, 4-Difluorobenzylamide (Compound 9).

¹H-NMR (ACETONE-*d*₆) δ 2.78 (s, 3 H), 4.64 (d, *J* = 6.1 Hz, 2 H), 5.38 (s, 2 H), 6.34 - 6.37 (m, 2 H), 6.74 (dd, *J* = 8.8, 2.3 Hz, 1 H), 7.23-7.45 (m, 6 H), 7.81 (s, 1 H).

### Example 10

### 1-Furan-2-ylmethyl-5-hydroxy-2-methyl-1H-indole-3-carboxylic Acid, 2, 5-Difluorobenzylamide (Compound 10).

¹H-NMR (ACETONE-*d*₆) δ 2.77 (s, 3 H), 4.68 (d, *J* = 6.1 Hz, 2 H), 5.38 (s, 2 H), 6.34 - 6.39 (m, 2 H), 6.77 (dd, *J* = 8.8, 2.3 Hz, 1 H), 7.04 -7.33 (m, 3 H), 7.39 (d, *J* = 16.1 Hz, 2 H), 7.45 (d, *J* = 2.6 Hz, 1 H), 7.88 (b s, 1 H).

### Example 11

### 5-Hydroxy-2-methyl-1-thiophen-2-ylmethyl-1H-indole-3-carboxylic Acid, 3, 5-Difluorobenzylamide (Compound 11).

¹H-NMR (ACETONE-*d*₆) δ 2.78 (s, 3 H), 4.66 (d, *J* = 6.2 Hz, 2 H), 5.59 (s, 2 H), 6.74 (dd, *J* = 8.8, 2.4 Hz, 1 H), 6.80-7.02 (m, 3 H), 7.08 (d, *J* = 8.8Hz, 1 H), 7.28 (d, *J* = 2.4 Hz, 1 H), 7.32 (d, *J* = 5.0 Hz, 1 H), 7.38 (d, *J* = 16 Hz, 2 H), 7.42 (b s, 1 H).

### Example 12

### 1-Furan-2-ylmethyl-5-hydroxy-2-methyl-1H-indole-3-carboxylic Acid 3, 5-Difluorobenzylamide (Compound 12).

¹H-NMR (ACETONE-*d*₆) δ 2.78 (s, 3 H), 4.66 (d, *J* = 6.2 Hz, 2 H), 5.38 (s, 2 H), 6.34 - 6.39 (m, 2 H), 6.74 (dd, *J* = 8.8, 2.4 Hz, 1 H), 6.80-6.90 (m, 1 H), 7.08 (dd, *J* = 8.8, 2.4 Hz, 1 H), 7.23-7.32 (m, 3 H), 7.27 (d, *J* = 2.4 Hz, 1 H) 7.42 (d, *J* = 15.9 Hz, 2 H), 7.45 (d, *J* = 2.1 Hz, 1 H), 7.84 (s, 1 H).

### Example 13

### 1-Benzyl-5-hydroxy-2-methyl-1H-indole-3-carboxylic Acid. 3, 4-Difluoro-benzylamide (Compound 13).

¹H-NMR (METHANOL-*d*₄) δ 2.55 (s, 3 H), 4.57 (s, 2 H), 5.38 (s, 2 H), 6.69 (dd, *J* = 2.2, 8.8 Hz, 1 H), 6.99 (2 br d, 2 H), 7.16 (d, *J* = 8.8 Hz, 1 H), 7.17-7.30 (m, 7 H).

### Example 14

### 5-Hydroxy-2-methyl-1-thiophen-2-ylmethyl-1H-indole-3-carboxylic Acid, 3-Fluorobenzylamide (Compound 14).

¹H-NMR (METHANOL-*d*₄) δ 2.65 (s, 3 H), 4.60 (s, 2 H), 5.52 (s, 2 H), 6.73 (dd, *J* = 2.2, 8.8 Hz, 1 H), 6.90-7.00 (m, 3 H), 7.10-7.39 (m, 6 H).

### Example 15

### 5-Hydroxy-2-methyl-1-thiophen-2-ylmethyl-1H-indole-3-carboxylic Acid, Benzylamide (Compound 15).

¹H-NMR (METHANOL-*d*₄) δ 2.64 (s, 3 H), 4.60 (s, 2 H), 5.52 (s, 2 H), 6.72 (dd, *J* = 2.2, 8.8 Hz, 1 H), 6.91 (2 br d, 2 H), 7.16 (d, *J* = 2.2 Hz, 1 H), 7.24-7.27 (m, 2 H), 7.31 (d, *J* = 4.0 Hz, 1 H), 7.35 (d, *J* = 7.0 Hz, 2 H), 7.42 (d, *J* = 7.5 Hz, 2 H).

### Example 16

### 5-Hydroxy-2-methyl-1-thiophen-2-ylmethyl-1H-indole-3-carboxylic Acid, 2-Fluorobenzylamide (Compound 16).

¹H-NMR (METHANOL-*d*₄) δ 2.67 (s, 3 H), 4.69 (s, 2 H), 5.55 (s, 2 H), 6.72 (dd, *J* = 2.6, 8.8 Hz, 1 H), 6.94 (m, 2 H), 7.40 (m, 1 H), 7.20 (m, 2 H), 7.28 (m, 1 H), 7.32 (overlap m, 1 H), 7.32 (d, *J* = 8.8 Hz, 1 H), 7.50 (t, *J* = 7.5 Hz, 1 H).

### Example 17

### 5-Hydroxy-2-methyl-1-thiophen-2-ylmethyl-1H-indole-3-carboxylic Acid, 3-Methoxybenzylamide (Compound 17).

¹H-NMR (METHANOL-*d*₄) δ 2.68 (s, 3 H), 3.81 (s, 3 H), 4.61 (s, 2 H), 5.55 (s, 2 H), 6.75 (dd, *J*= 2.6, 8.8 Hz, 1 H), 6.85 (dd, *J*= 2.5, 8.4 Hz, 1 H), 6.94 (m, 2 H), 7.02 (2 br d, 2 H), 7.19(d, *J* = 2.5 Hz, 1 H), 7.28 (m, 2 H), 7.32 (d, *J* = 8.7 Hz, 1 H).

### Example 18

### 1-Butyl-5-hydroxy-2-methyl-1H-indole-3-carboxylic Acid, 3-Methoxy-benzylamide (Compound 18).

¹H-NMR (METHANOL-*d*₄) δ 0.95 (t, *J* = 7.5 Hz, 3 H), 1.39 (m, 2 H), 1.71 (m, 2 H), 2.60 (s, 3 H), 3.78 (s, 3 H), 4.11 (t, *J* = 7.5 Hz, 2 H), 4.58 (s, 2 H), 6.72 (dd, *J* = 2.2, 8.8 Hz, 1 H), 7.03 (dd, *J* = 2.2, 8.4 Hz, 1 H), 6.90 (2 br d, 2 H), 7.15 (d, *J* = 2.2 Hz, 1 H), 7.21 (d, *J*= 8.8 Hz, 1 H), 7.26 (d, *J*= 8.4 Hz, I H).

### Example 19

### 5-Hydroxy-2-methyl-1-thiophen-2-ylmethyl-1H-indole-3-carboxylic Acid, 4-Fluorobenzylamide (Compound 19).

¹H-NMR (METHANOL-*d*₄) δ 2.63 (s, 3 H), 4.58 (s, 2 H), 5.53 (s, 2 H), 6.72 (dd, *J* = 2.6, 8.8 Hz, 1 H), 6.91 (2 br d, 2 H), 7.06 (t, *J*= 8.8 Hz, 2 H), 7.15 (d, *J*= 2.2 Hz, 1 H), 7.25 (dd, *J* = 4.0, 6.6 Hz, 1 H), 7.29 (d, *J* = 8.8 Hz, 1 H), 7.35 (dd, *J*= 13.6, 8.4 Hz, 2 H).

### Example 20

### 5-Hydroxy-2-methyl-1-thiophen-2-ylmethyl-1H-indole-3-carboxylic Acid, 4-Methylbenzylamide (Compound 20).

¹H-NMR (METHANOL-*d*₄) δ 2.32 (s, 3 H), 2.64 (s, 3 H), 4.55 (s, 2 H), 5.52 (s, 2 H), 6.73 (dd, *J* = 2.6, 8.8 Hz, 1 H), 6.91 (m, 2 H), 7.14 (d, *J* = 2.2 Hz, 1 H), 7.15 (d, *J* = 9 Hz, 2 H), 7.24 - 7.30 (m, 4 H).

### Example 21

### 5-Hydroxy-2-methyl-1-thiophen-2-ylmethyl-1H-indole-3-carboxylic Acid, 3-Chlorobenzylamide (Compound 21).

¹H-NMR (METHANOL-*d*₄) δ 2.65 (s, 3 H), 4 58 (s, 2 H), 5 53 (s, 2 H), 6.72 (dd, *J* = 2.6, 8.8 Hz, 1 H), 6 91 (2 br d, 2 H), 7.16 (d, *J* = 2 2 Hz, 1 H), 7 24-7.34 (m, 5 H), 7 42 (s, 1 H).

### Example 22

### 5-Hydroxy-2-methyl-1-thiophen-2-ylmethyl-1H-indole-3-carboxylic Acid, 4-Chlorobenzylamide (Compound 22).

¹H-NMR (METHANOL-*d*₄) δ 2.64 (s, 3 H), 4 57 (s, 2 H), 5.52 (s, 2 H), 6.72 (dd, *J* = 2.6, 8.8 Hz, 1 H), 6.91 (2 br dd, 2 H), 7.15 (d, *J* = 2.2 Hz, 1 H), 7.25 (dd, *J* = 2.2, 4.0 Hz, 1 H), 7 29 (d, *J*= 9 Hz, 1 H), 7.35 (d, *J*= 8.4 Hz, 2 H), 7.40 (d, *J*= 8.4 Hz, 2 H).

### Example 23

### 5-Hydroxy-2-methyl-1-thiophen-2-ylmethyl-1H-indole-3-carboxylic Acid, 2-methoxybenzylamide (Compound 23).

¹H-NMR (METHANOL-*d*₄) δ 2 64 (s, 3 H), 3.91 (s, 3 H), 4.60 (s, 2 H), 5.52 (s, 2 H), 6.72 (dd, *J* = 2.6, 8.8 Hz, 1 H), 6.88-6.95 (m, 3 H), 7.00 (d, *J* = 8.0 Hz, 1 H), 7.14 (d, *J* = 2.2 Hz, 1 H), 7.24-7.35 (m, 4 H).

### Example 24

### 5-Hydroxy-2-methyl-1-thiophen-2-ylmethyl-1H-indole-3-carboxylic Acid, 3-Trifluoromethylbenzylamide (Compound 24)

¹H-NMR (METHANOL-*d*₄) δ 2 64 (s, 3 H), 4.67 (s, 2 H), 5.53 (s, 2 H), 6.73 (dd, *J* = 2.6, 8.8 Hz, 1 H), 6.91 (m, 2 H), 7 17 (d, *J* = 2.2 Hz, 1 H), 7.25 (dd, *J*= 22, 4.0 Hz, 1 H), 7 30 (d, *J* = 9.0 Hz, 1 H), 7.53 (m, 2 H), 7.70 (m, 2 H)

### Example 25

### 1-Benzyl-2-ethyl-5-hydroxy-1H-indole-3-carboxylic Acid, 3,4-Difluorobenzylamide (Compound 25).

¹H-NMR (METHANOL-*d*₄) δ 1.13 (t, *J* = 7.5 Hz, 3 H), 3.04 (q, *J =* 7.5 Hz, 2 H), 4.58 (s, 2 H), 5.41 (s, 2 H), 6.64 (dd, *J*= 2.6, 8.8 Hz, 1 H), 6.98 (2 br d, 2 H), 7.10 (d, *J*= 8.8 Hz, 1 H), 7.17 (d, *J*= 2.2 Hz, 1 H), 7.20-7.35 (m, 6 H).

### Example 26

### 1-Benzyl-2-ethyl-5-hydroxy-1H-indole-3-carboxylic Acid, 3-Methoxy-benzylamide (Compound 26).

¹H-NMR (METHANOL-*d*₄) δ 1.13 (t, *J*= 7.5 Hz, 3 H), 3.04 (q, *J*= 7.5 Hz, 2 H), 3.78 (s, 3 H), 4.60 (d, *J*= 6.2 Hz, 2 H), 5.39 (s, 2 H), 6.64 (dd, *J*= 2.6, 8.8 Hz, 1 H), 6.80 (br d, 1 H), 6.98 (2br d, 4 H), 7.08 (d, *J* = 8.8 Hz, 1 H), 7.17 (d, *J*= 2.2 Hz, 1 H), 7.22-7.25 (m, 4 H).

### Example 28

### 1-Benzyl-5-hydroxy-2-isopropyl-1H-indole-3-carboxylic Acid, 3,4-Difluorobenzamide (Compound 28).

¹H-NMR (CHLOROFORM-*d*) δ 1.34 (d, *J* = 7.0 Hz, 6 H), 3.57-3.74 (m, 1 H), 4.51 (d, *J*= 5.9 Hz, 2 H), 5.36 (s, 2 H), 6.38 (t, *J* = 6.0 Hz, 1 H), 6.68 (dd, *J*= 8.8, 2.3 Hz, 1 H), 6.87-6.95 (m, 3 H), 6.97-7.05 (m, 2 H), 7.05-7.16 (m, 2 H), 7.17-7.28 (m, 3 H).

### Example 32

### 1-Benzyl-2-ethyl-5-hydroxy-1H-indole-3-carboxylic Acid 3,5-Difluorobenzylamide (Compound 32).

¹H-NMR (METHANOL-*d*₄) δ 1.13 (t, *J* = 7.5 Hz, 3 H), 3.05 (q, *J*= 7.5 Hz, 2 H), 4.61 (s, 2 H), 5.41 (s, 2 H), 6.68 (dd, *J* = 2.2, 8.8 Hz, 1 H), 6.82 (m, 1 H), 7.00 (m, 4 H), 7.11 (d, *J* = 8.8 Hz, 1 H), 7.18-7.30 (m, 4 H).

### Example 33

### 1-Benzyl-5-hydroxy-2-isopropyl-1H-indole-3-carboxylic Acid, 3,5-difluorobenzylamide (Compound 33).

¹H-NMR (CHLOROFORM-*d*) δ 1.39 (d, *J* = 7.3 Hz, 6 H), 3.65-3.79 (m, 1 H), 4.68 (d, *J* = 6.2 Hz, 2 H), 5.42 (s, 2 H), 6.32 (t, *J* = 6.0 Hz, 1 H), 6.66-6.77 (m, 2 H), 6.89-6.98 (m, 4 H), 7.01 (d, *J* = 8.8 Hz, 1 H), 7.13 (d, *J* = 2.1 Hz, 1 H), 7.21-7.34 (m, 3 H).

### Example 34

### 1-Benzyl-5-hydroxy-2-isopropyl-1H-indole-3-carboxylic Acid, 3-methoxybenzylamide (Compound 34).

¹H-NMR (CHLOROFORM-*d*) δ 1.38 (d, *J*= 7.0 Hz, 6 H), 3.80 (s, 3 H), 4.67 (d, *J* = 5.9 Hz, 2 H), 5.39 (s, 2 H), 6.22 (t, *J* = 5.6 Hz, 1 H), 6.67 (dd, *J* = 8.6, 2.5 Hz, 1 H), 6.79-6.85 (m, 1 H), 6.89-7.02 (m, 5 H), 7.11 (d, *J* = 2.3 Hz, 1 H), 7.20-7.32 (m, 4 H).

### Example 48

### 1-Benzyl-5-hydroxy-2-phenyl-1H-indole-3-carboxylic Acid, 3,5-Difluorobenzylamide (Compound 48)

1H NMR (METHANOL -*d*) δ 4.39 (s, 2 H), 5.23 (s, 2 H), 6.67 (2d, *J* = 8.4 Hz, 2 H), 6.79 (m, 2 H), 6.90 (2 d, 8.4 Hz, 2 H), 7.17 (d, *J* = 8.4 Hz, 1 H), 7.22 (m, 3 H), 7.39 - 7.47 (m, 6 H).

**The following compounds were prepared, by the General Procedures illustrated in Schemes 2 and 3, below, from ethyl 1-benzyl-2-methyl-1H-indole-3-carboxylate (Compound 57), which was synthesized as described in**

### General Procedure 11:

### Example 36

### 1-Benzyl-2-methyl-1H-indole-3-carboxylic Acid, 3,5-Difluorobenzylamide (Compound 36)

¹H-NMR (CHLOROFORM-*d*) δ 2.72 (s, 3 H), 4.71 (d, *J*= 3.9 Hz, 2 H), 5.37 (s, 2 H), 6.72 (dt, *J* = 2.6, 8.8 Hz, 1 H), 6.97 (br dd, 3 H), 7.19-7.30 (m, 6 H), 7.72 (d, *J* = 7.0 Hz, 1 H).

### Example 37

### 1-Benzyl-2-methyl-1H-indole-3-carboxylic Acid, 3,4-Difluorobenzylamide (Compound 37).

¹H-NMR (CHLOROFORM-*d*) δ 2.72 (s, 3 H), 4.68 (d, *J* = 6.1 Hz, 2 H), 5.37 (s, 2 H), 6.33 (br s, 1 H), 6.99 (br d, 2 H), 7.14-7.30 (m, 9 H), 7.70 (d, *J* = 6.6 Hz, 1 H).

### Example 38

### 1-Benzyl-2-methyl-1H-indole-3-carboxylic Acid, 3-Fluorobenzylamide (Compound 38)

¹H-NMR (CHLOROFORM-*d*) δ 2.73 (s, 3 H), 4.73 (d, *J* = 5.7 Hz, 2 H), 5.37 (s, 2 H), 6.32 (br s, 1 H), 6.99 (br d, 3 H), 7.12-7.36 (m, 9 H), 7.72 (d, *J*= 6.6 Hz, 1 H).

### Example 39

### 1-Benzyl-2-methyl-1H-indole-3-carboxylic Acid, 3-Methoxybenzylamide (Compound 39).

¹H-NMR (CHLOROFORM-*d*) δ 2.72 (s, 3 H), 3.82 (s, 3 H), 4.71 (d, *J* = 5.8 Hz, 2 H), 5.36 (s, 2 H), 6.27 (br s, 1 H), 6.85 (dd, *J* = 2.4. 8.8 Hz, 1 H), 7.00 (br d, 3 H), 7.17 (m, 2 H), 7.26-7.32 (m, 6 H), 7.72 (m, 1 H).

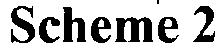

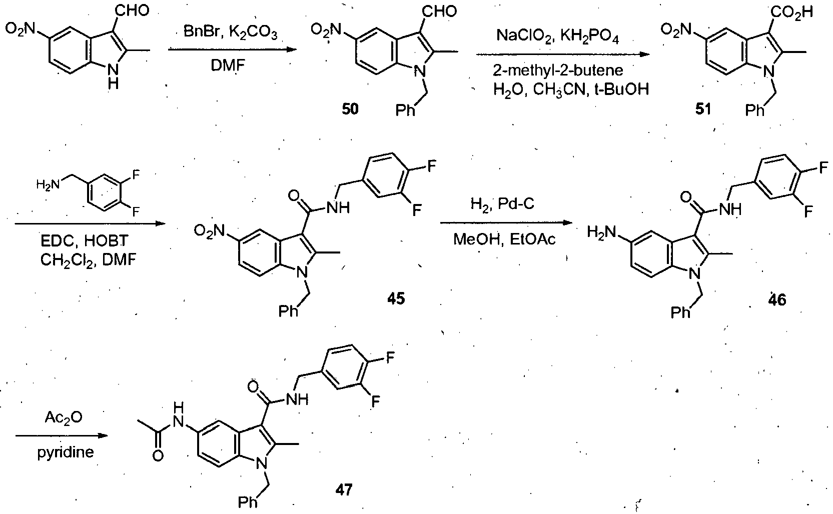

### Example 50

### 1-Benzyl-2-methyl-5-nitro-1H-indole-3-carboxaldehyde (Compound 50).

**General Procedure 4.** To a solution of 2-methyl-5-nitro-1H-indole-3-carboxaldehyde (500 mg, 2.45 mmol) in DMF (5 ml) was added potassium carbonate (1.0 g, 7.35 mmol) and benzyl bromide (0.44 ml, 3.68 mmol). The mixture was stirred at room temperature for 4 h, diluted with EtOAc, washed with H₂O, brine, dried over Na₂SO₄, and concentrated under reduced pressure. The residue was triturated with Et₂O-hexane to yield 1-benzyl-2-methyl-5-nitro-1H-indole-3-carboxaldehyde (**Compound 50**) as a yellow solid (600 mg, 83%).
¹H-NMR (METHANOL-*d*₄) δ 2.76 (s, 3 H), 5.60 (s, 2 H), 7.04-7.11 (m, 2 H), 7.26-7.37 (m, 3 H), 7.60 (d, *J* = 9.1 Hz, 1 H), 8.15 (dd, *J* = 8.9, 2.2 Hz, 1 H), 9.11 (d, *J* = 2.3 Hz, 1 H), 10.20 (s, 1 H).

### Example 51

### 1-Benzyl-2-methyl-5-nitro-1H-indole-3-carboxylic Acid (Compound 51).

**General Procedure 5.** To a suspension of 1-benzyl-2-methyl-5-nitro-1H-indole-3-carboxaldehyde (**Compound 50**, 150 mg, 0.51 mmol) in acetonitrile (6 ml), *tert-*butanol (6 ml) and H₂O (6 ml) was added 2-methyl-2-butene (4 ml), potassium phosphate monobasic (1.4 g, 10.2 mmol), sodium chlorite (80%, 1.15 g, 10.2 mmol). The mixture was stirred at room temperature for 20 h, more potassium phosphate monobasic (0.35 g, 2.6 mmol) and sodium chlorite (80%, 0.29 g, 2.6 mmol) were added and stirred at room temperature for 24 h. The solvent was removed under reduced pressure. The residue solid was washed with H₂O (×3) and filtered, dissolved in acetone and filtered to yield 1-benzyl-2-methyl-5-nitro-1H-indole-3-carboxylic acid (**Compound 51**) as a yellow powder (160 mg, 100%). ¹H-NMR (ACETONE-*d*₆) δ 2.83 (s, 3 H), 5.68 (s, 2 H), 7.06-7.15 (m, 2 H), 7.25-7.41 (m, 3 H), 7.68 (d, *J* = 9.1 Hz, 1 H), 8.10 (dd, *J =* 9.1, 2.3 Hz, 1 H), 9.11 (d, *J =* 2.3 Hz, 1 H).

### Example 45

**1-Benzyl-2-methyl-5-nitro-1H-indole-3-carboxylic Acid, 3,4-Difluorobenzylamide (Compound 45) -** The title compound was prepared from 1-benzyl-2-methyl-5-nitro-1H-indole-3-carboxylic acid (**Compound 51**) by General Procedure 3.
¹H-NMR (DMSO-*d*₆) δ 2.60 (s, 3 H), 4.51 (d, *J*= 6.2 Hz, 2 H), 5.60 (s, 2 H), 7.01-7.08 (m, 2 H), 7.20-7.49 (m, 6 H), 7.74 (d, *J* = 8.8 Hz, 1 H), 8.05 (dd, *J* = 9.1, 2.3 Hz, 1 H), 8.57 (t, *J* = 5.7 Hz, 1 H), 8.71 (d, *J* = 2.1 Hz, 1 H).

### Example 52

**1-Benzyl-5-(benzyloxy)-1H-indole-3-carboxylic Acid, N-(3,4-difluorobenzyl)-**(**Compound 52**) - The title compound was prepared from 5-(benzyloxy)-1H-indole-3-carboxaldehyde by, in order, General Procedures 4, 5, and 3.
¹H-NMR (METHANOL-*d*₄) δ 4.53 (s, 2 H), 5.11 (s, 2 H), 5.39 (s, 2 H), 6.92 (dd, *J* = 9.1, 2.3 Hz, 1 H), 7.14-7.39 (m, 12 H), 7.43-7.49 (m, 2 H), 7.79 (d, *J*= 2.3 Hz, 1 H), 7.91 (s, 1 H).

### Example 46

**5-Amino-1-benzyl-2-methyl-1H-indole-3-carboxylic Acid, 3,4-Difluorobenzylamide (Compound 46). General Procedure 6.** To a solution of 1-benzyl-2-methyl-5-nitro-1H-indole-3-carboxylic acid, 3,4-difluorobenzylamide (Compound 45, 97 mg, 0.22 mmol) in MeOH (20 ml) and EtOAc (20 ml) was added Pd-C (10 %, 47 mg, 0.045 mmol). The reaction was stirred under hydrogen for 24 h, filtered through Celite, washed with MeOH-EtOAc (1:1) to yield 5-amino-1-benzyl-2-methyl-1H-indole-3-carboxylic acid, 3,4-difluorobenzylamide (**Compound 46**) as a white solid (93 mg, 100%).
¹H-NMR (METHANOL-*d*₄) δ 2.54 (s, 3 H), 4.58 (s, 2 H), 5.35 (s, 2 H), 6.69 (dd, *J* = 8.5, 2.1 Hz, 1 H), 6.95-7.01 (m, 2 H), 7.12 (d, *J* = 8.5 Hz, 1 H), 7.16-7.36 (m, 6 H).

### Example 27 1-Benzyl-5-hydroxy-1H-indole-3-carboxylic Acid, 3,4-Difluorobenzylamide

(**Compound 27**) The title compound was prepared from 1-benzyl-5-(benzyloxy)-1H-indole-3-carboxylic acid, 3,4-difluorobenzylamide (Compound 52) by General Procedure 6.
¹H-NMR (METHANOL-*d*₄) δ 4.52 (s, 2 H), 5.36 (s, 2 H), 6.74 (dd, *J* = 8.8, 2.6 Hz, 1 H), 7.12-7.36 (m, 9 H), 7.54 (d, *J* = 2.1 Hz, 1 H), 7.86 (s, 1 H).

### Example 47

**5-Acetamido-1-benzyl-2-methyl-1H-indole-3-carboxylic Acid, 3,4-Difluorobenzylamide (Compound 47). General Procedure** 7. To a solution of 5-amino-1-benzyl-2-methyl-1H-indole-3-carboxylic acid, 3,4-difluorobenzylamide (**Compound 46**, 50 mg, 0.12 mmol) in pyridine (3 ml) was added acetic anhydride (120 µl, 1.23 mmol). The reaction was stirred at room temperature for 72 h, diluted with EtOAc, washed successively with 1M HCl, H₂O, brine, dried over Na₂SO₄, and concentrated under reduced pressure. The residue was crystallized from CH₂Cl₂-Et₂O to yield 5-acetamido-1-benzyl-2-methyl-1H-indole-3-carboxylic acid, 3,4-difluorobenzylamide (**Compound 47**) as a white solid (37 mg, 68%). ¹H-NMR (METHANOL-*d*₄) δ 2.13 (s, 3 H), 2.58 (s, 3 H), 4.59 (s, 2 H), 5.44 (s, 2 H), 6.96-7.04 (m, 2 H), 7.14-7.37 (m, 9 H), 7.99 (d, *J* = 2.1 Hz, 1 H).

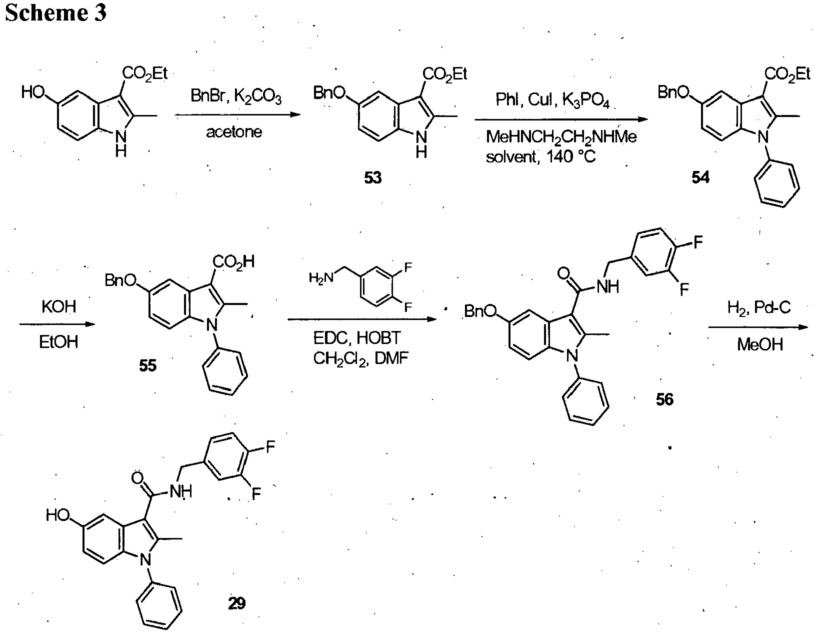

### Example 53

**5-Benzyloxy-2-methyl-1H-indole-3-carboxylic Acid, Ethyl Ester (Compound 53). General Procedure 8.** To a mixture of 5-hydroxy-2-methyl-1H-indole-3-carboxylic acid, ethyl ester (0.76 g, 3.47 mmol) and potassium carbonate (0.92 g, 6.67 mmol) in acetonitrile (10 ml) was added benzyl bromide (1.0 ml, 1.4 g, 8.4 mmol). The mixture was heated at 75-80 °C for 18 h. The reaction was cooled to room temperature, quenched with water, extracted with EtOAc, washed with brine, dried over Na₂SO₄, and concentrated under reduced pressure. The residue was purified by flash column chromatography on silica gel (30% EtOAc-hexanes) to yield 5-benzyloxy-2-methyl-1H-indole-3-carboxylic acid, ethyl ester (**Compound 53**) as a yellow solid (0.56 g, 52%). ¹H-NMR (METHANOL-*d*₄) δ 1.39 (t, *J* = 7.0 Hz, 3 H), 2.64 (s, 3 H), 4.32 (q, *J* = 7.0 Hz, 2 H), 5.10 (s, 2 H), 6.84 (dd, *J*= 2.2, 8.8 Hz, 1 H), 7.20 (d, *J* = 8.8 Hz, 1 H), 7.23-7.40 (m, 5 H), 7.45 (2 br d, 2 H), 7.58 (d, 2.2 Hz, 1 H).

### Example 54

### 5-Benzyloxy-2-methyl-1-phenyl-1H-indole-3-carboxylic Acid, Ethyl Ester (Compound 54).

General Procedure 9. To a mixture of 5-benzyloxy-2-methyl-1H-indole-3-carboxylic acid, ethyl ester (**Compound 53**, 0.14 g, 0.45 mmol) in toluene (6 ml) having been degassed under argon for 15 min. was added 2-iodo-benzene (0.10 ml, 0.48 g, 0.88 mmol), potassium phosphate (0.20 g, 0.94 mmol), copper (I) iodide (24 mg, 0.13 mmol), and then N,N'-dimethylethylenediamine (12 mg, 0.14 mmol) with continued degassing. The tube was then sealed and mixture was heated at 140 °C for 24 h. The reaction was then cooled and filtered. The filtrate was concentrated under reduced pressure and the crude product residue was purified by flash column chromatography on silica gel (30% EtOAc-hexanes) to yield 5-benzyloxy-2-methyl-1-phenyl-1H-indole-3-carboxylic acid, ethyl ester (**Compound 54**) as an orange oil (0.13 g, 76%).
¹H-NMR (CHLOROFORM-*d*) δ 1.44 (t, *J* = 7.0 Hz, 3 H), 2.57 (s, 3 H), 4.42 (q, *J* = 7.0 Hz, 2 H), 5.16 (s, 2 H), 6.86 (dd, *J* = 2.7, 8.8 Hz, 1 H), 6.92 (d, *J* = 8.8 Hz, 1 H), 7.25-7.41 (m, 5 H), 7.48-7.60 (m, 5 H), 7.78 (d, *J*= 2.7 Hz, 1 H).

### Example 55

**5-Benzyloxy-2-methyl-1-phenyl-1H-indole-3-carboxylic Acid (Compound 55).** The title compound was prepared from 5-benzyloxy-2-methyl-1-phenyl-1H-indole-3-carboxylic acid, ethyl ester (**Compound 54**) by General Procedure 2. ¹H-NMR (METHANOL-*d*₄) δ 2.60 (s, 3 H), 5.12 (s, 2 H), 6.84 (dd, *J* = 2.6, 8.8 **Hz, 1 H), 6.90 (d, *J*= 8.8 Hz, 1 H), 7.26-7.63 (m, 10 H), 7.77 (d, *J*= 2.6 Hz, 1 H).**

### Example 56

**5-Benzyloxy-2-methyl-1-phenyl-1H-indole-3-carboxylic Acid, 3,4-difluorobenzylamide (Compound 56).** The title compound was prepared from 5-benzyloxy-2-methyl-1-phenyl-1H-indole-3-carboxylic acid (Compound 55) by General Procedure 3.
¹H-NMR (METHANOL-*d*₄) δ 2.44 (s, 3 H), 4.60 (s, 2 H), 5.10 (s, 2 H), 6.85 (dd, *J* = 2.2, 8.8 Hz, 1 H), 6.90 (d, *J* = 8.8 Hz, 1 H), 7.21-7.45 (m, 8 H), 7.54-7.66 (m, 3 H).

### Example 29

**5-Hydroxy-2-methyl-1-phenyl-1H-indole-3-carboxylic Acid 3,4-Difluorobenzylamide (Compound 29). General Procedure 10.** To a mixture of 5-benzyloxy-2-methyl-1-phenyl-1H-indole-3-carboxylic acid, 3,4-difluorobenzylamide (**Compound 56**, 0.15 g, 0.31 mmol) in methanol (15 ml), which was degassed with argon for 10 min, was added 10% palladium on carbon (0.17 g), with continued degassing. The reaction was placed in par tube on hydrogenator and hydrogenated at 45 psi for 18 h. The reaction was then filtered, concentrated under reduced pressure and the crude product residue was purified by flash column chromatography on silica gel (30% EtOAc-hexanes) to yield 5-hydroxy-2-methyl-1-phenyl-1H-indole-3-carboxylic acid, 3,4-difluorobenzylamide (**Compound 29**) as a solid (0.11 g, 92%).
¹H-NMR (METHANOL-*d*₄) δ 2.42 (s, 3 H), 4.59 (s, 2 H), 6.66 (dd, *J* = 2.2, 8.8 Hz, 1 H), 6.82 (d, *J* = 8.8 Hz, 1 H), 7.21-7.26 (m, 3 H), 7.30-7.40 (m, 3 H), 7.53-7.65 (m, 3 H).

### Example 30

**5-Hydroxy-2-methyl-1-pyridin-2-yl-1H-indole-3-carboxylic Acid 3,4-Difluorobenzylamide (Compound 30)** - The title compound was prepared from 2-iodo-pyridine by following, in order, General procedures 8, 9, 2, 3 and 10.
¹H-NMR (METHANOL-*d*₄) δ 2.51 (s, 3 H), 4.59 (s, 2 H), 6.70 (dd, *J* = 2.6, 8.8 Hz, 1 H), 7.03 (d, *J* = 8.8 Hz, 1 H), 7.20-7.26 (m, 3 H), 7.33 (m, 1 H), 7.55 (m, 2 H), 8.10 (dt, *J* = 2.2, 8.8 Hz, 1 H), 8.65 (dd, *J* = 2.2, 5.7 Hz, 1 H).

### Example 31

**5-Hydroxy-2-methyl-1-thiophen-2-yl-1H-indole-3-carboxylic Acid 3,4-Difluorobenzylamide (Compound 31)** - The title compound was prepared from 2-iodothiophene by following, in order, General Procedures 8, 9, 2, 3 and 10.
¹H-NMR (METHANOL-*d*₄) δ 2.45 (s, 3 H), 4.58 (s, 2 H), 6.70 (dd, *J* = 2.6, 8.8 Hz, 1 H), 6.94 (d, *J* = 8.8 Hz, 1 H), 7.12 (dd, *J* = 1.3, 3.5 Hz, 1 H), 7.16-7.46 (m, 5 H), 7.55 (dd, *J* = 1.4, 5.7 Hz, 1 H).

### Example 35

**5-Methoxy-2-methyl-1-phenyl-1H-indole-3-carboxylic Acid, 3,5-Difluorobenzylamide (Compound 35)** - The title compound was prepared from methyl iodide by following, in order, General Procedures 8, 9, 2 and 3.
¹H-NMR (CHLOROFORM-*d*) δ 2.55 (s, 3 H), 3.85 (s, 3 H), 4.72 (d, *J*= 6.1 Hz , 2 H), 6.25 (br s, 1 H), 6.73 (m, 1 H), 6.80 (dd, *J* = 2.2, 8.8 Hz, 1 H), 6.97 (2d, *J* = 8.8 Hz, 3 H), 7.26-7.33 (m, 3 H), 7.51-7.60 (m, 3 H).

### Example 40

### 5-Methoxy-1,2-dimethyl-1H-indole-3-carboxylic Acid, 3,5-difluorobenzylamide (Compound 40) - The title compound was prepared from methyl iodide by following, in order, General procedures 8, 9, 2 and 3.

¹H-NMR (CHLOROFORM-*d*) δ 2.73 (s, 3 H), 3.69 (s, 3 H), 3.84 (s, 3 H), 4.69 (d, *J* = 6.1 Hz, 2 H), 6.19 (br s, 1 H), 6.71 (dt, *J* = 2.2, 8.8 Hz, 1 H), 6.91 (dd, *J* = 2.2, 8.8 Hz, 1 H), 6.95 (br d, 2 H), 7.19 (d, *J*= 2.2 Hz, 1 H), 7.21-7.26 (m, 1 H).

### Example 41

**5-Methoxy-1,2-dimethyl-1H-indole-3-carboxylic Acid 3-Fluorobenzylamide (Compound 41) -** The title compound was prepared from methyl iodide by following, in order, General Procedures 8, 9, 2 and 3.
¹H-NMR (CHLOROFORM-*d*) δ 2.73 (s, 3 H), 3.68 (s, 3 H), 3.81 (s, 3 H), 4.71 (d, *J* = 6.1 Hz, 2 H), 6.15 (br s, 1 H), 6.87 (dd, *J* = 2.2, 8.8 Hz, 1 H), 6.97 (m, 2 H), 7.13 (2 br d, 1 H), 7.18 (d, *J* = 2.6 Hz, 1 H), 7.22 (d, *J* = 8.4 Hz, 2 H).

### Example 49

**5-Benzyloxy-2-methyl-1-pyridin-2-yl-1H-indole-3-Carboxylic Acid, 3,4-Difluorobenzylamide (Compound 49) -** The title compound was prepared from 2-iodopyridine by following, in order, General Procedures 8, 9, 2 and 3.
¹H-NMR (CHLOROFORM-*d*) δ 2.52 (s, 3 H), 4.60 (s, 2 H), 5.10 (s, 2 H), 6.89 (dd, *J* = 2.6, 8.8 Hz, 1 H), 7.10 (d, *J* = 8.8 Hz, 1 H), 7.21-7.45 (m, 6 H), 7.55 (2 br d, 2 H), 8.10 (dt, *J* = 2.2, 7.9 Hz, 1 H), 8.65 (m, 1 H).

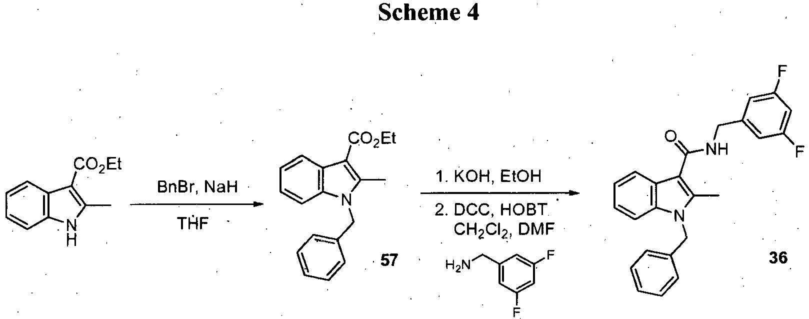

### Example 57

### 1-Benzyl-2-methyl-1H-indole-3-carboxylic Acid, Ethyl Ester (Compound 57).

**General Procedure 11. -** To a mixture of sodium hydride (0.28g, 60% in mineral oil, 0.17g, 7.0 mmol) in 10 ml of tetrahydrofuran stirring at 0 °C under argon, was added 2-methyl-1H-indole-3-carboxylic acid ethyl ester (1.17g, 5.8 mmol) and the solution was stirred at 0 °C for 15 min. Benzyl bromide (0.80 ml, 1.15 g, 6.7 mmol) was then added and the reaction allowed to warm to room temperature and stirred for 24 h. The reaction was cooled to 0 °C, quenched with water, extracted with EtOAc, washed with brine, dried over Na₂SO₄, and concentrated under reduced pressure. The residue was purified by flash column chromatography on silica gel (40% EtOAc-hexanes) to yield 1-benzyl-2-methyl-1H-indole-3-carboxylic acid, ethyl ester (Compound 57) as a tan solid (1.13 g, 67%).
¹H-NMR (CHLOROFORM-*d*) δ 1.46 (t, *J* = 7.0 Hz, 3 H), 2.73 (s, 3 H), 4.42 (q, *J* = 7.0 Hz, 2 H), 5.36 (s, 2 H), 6.97 (dd, *J =* 2.1, 8.8 Hz, 2 H), 7.15-7.30 (m, 6 H), 8.17 (d, *J* = 8.5 Hz, 1 H).

### Example 42

**2-Methyl-1-thiophen-2-ylmethyl-1H-indole-3-carboxylic Acid, 3,4-difluorobenzylamide (Compound 42) -** The title compound was prepared from 2-bromomethylthiophene by following, in order, General Procedures 11, 2 and 3. ¹H-NMR (CHLOROFORM-*d*) δ 2.81 (s, 3 H), 4.66 (s, 2 H), 5.49 (s, 2 H), 6.29 (br s, 1 H), 6.83 (br d, 1 H), 6.91 (m, 1 H), 7.12-7.26 (m, 4 H), 7.42 (m, 2 H), 7.68 (m, 2 H).

### Example 43

**2-Methyl-1-thiophen-2-ylmethyl-1H-indole-3-carboxylic Acid, 3-Methoxybenzylamide (Compound 43) -** The title compound was prepared from 2-bromomethylthiophene by following, in order, General Procedures 11, 2 and 3.
¹H-NMR (CHLOROFORM-*d*) δ 1.56 (s, 3 H), 2.81 (s, 2 H), 3.81 (s, 3 H), 4.69 (d, *J* = 5.7 Hz , 1 H), 5.48 (s, 2 H), 6.25 (br t, 1H), 6.72 (dd, *J* = 2.6, 8.8 Hz, 1 H), 6.88-6.95 (m, 3 H), 7.00 (d, *J* = 8.0 Hz, 1 H), 7.14 (d, *J* = 2.2 Hz, 1 H), 6.84 (br d, 2 H), 6.91 (m, 1 H), 6.99 (m, 2 H), 7.17-7.22 (m, 3 H), 7.30 (d, *J* = 8.0 Hz, 1 H), 7.39 (d, *J* = 7.0 Hz, 1 H), 7.68 (d, *J* = 2.2 Hz, 1 H).

### Example 44

**2-Methyl-1-thiophen-2-ylmethyl-1H-indole-3-carboxylic Acid 3,5-Difluorobenzylamide (Compound 44) -** The title compound was prepared from 2-bromomethylthiophene by following, in order, General Procedures 11, 2 and 3. ¹H-NMR (METHANOL-*d*₄) δ 2.63 (s, 3 H), 4.58 (s, 2 H), 5.53 (s, 2 H), 6.72 (dd, *J* = 2.6, 8.8 Hz, 1 H), 6.91 (2 br d, 2. H), 7.06 (t, *J* = 8.8 Hz, 2 H), 7.15 (d, *J* = 2.2 Hz, 1 H), 7.25 (dd, *J* = 4.0, 6.6 Hz, 1 H), 7.29 (d, *J* = 8.8 Hz, 1 H), 7.35 (dd, *J* = 13.6, 8.4 Hz, 2 H).

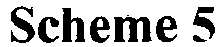

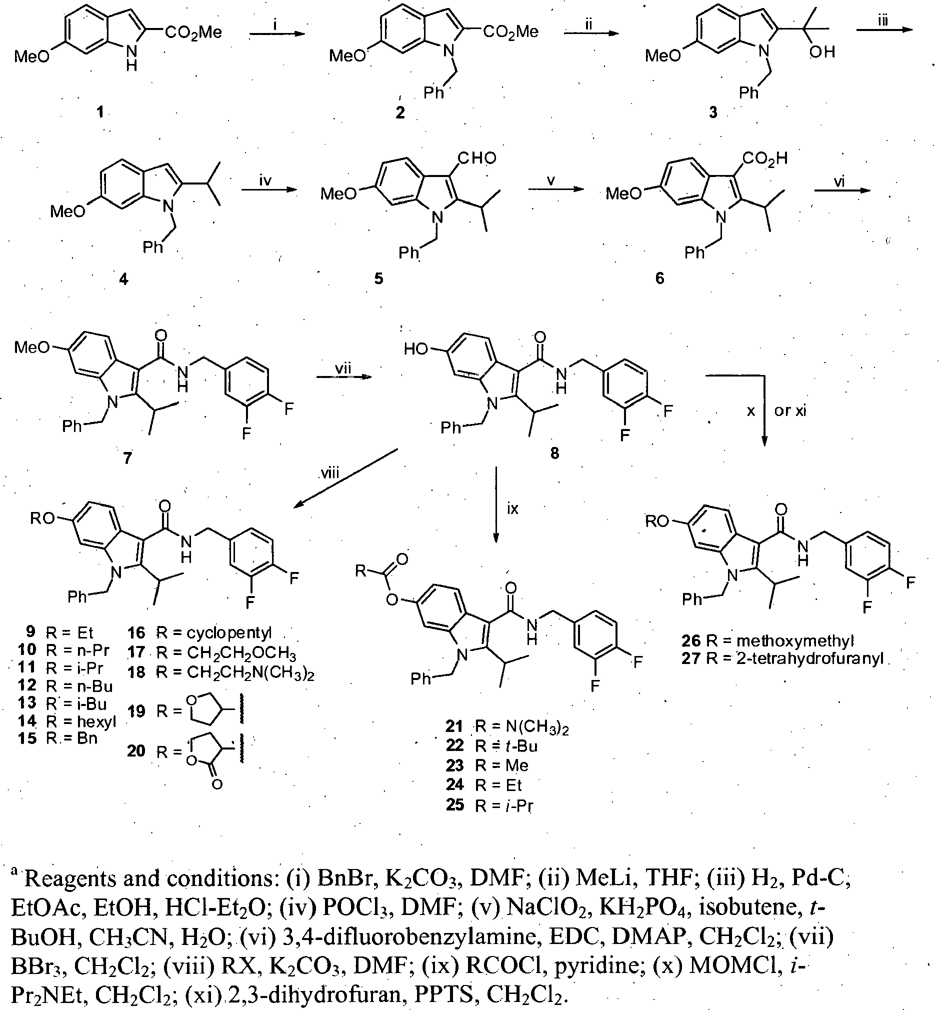

### Example 102

**Methyl 1-Benzyl-6-methoxy-1H-indole-2-carboxylate (Compound 102).** To a solution of methyl 6-methoxy-1H-indole-2-carboxylate (**Compound 101 or Compound 1, Scheme 5**), 1.0 g, 4.9 mmol) in DMF (10 ml) was added K₂CO₃ (2.0 g, 14.6 mmol) and benzyl bromide (0.87 ml, 7.3 mmol). The mixture was stirred at room temperature for 40 h and was diluted with EtOAc, washed with H₂O, brine, dried over Na₂SO₄ and concentrated *in vacuo.* The residue was purified by crystallization from Et₂O to yield the title compound as an off-white solid.
¹H NMR (500 MHz, CHLOROFORM-*d*) δ ppm 3.81 (s, 3 H), 3.85 (s, 3 H), 5.81 (s, 2 H), 6.73 (d, *J* = 2.0 Hz, 1 H), 6.84 (dd, *J* = 8.8, 2.0 Hz, 1 H), 7.07 (d, *J* = 6.8 Hz, 2 H), 7.19 - 7.29 (m, 3 H), 7.33 (s, 1 H), 7.58 (d, *J* = 8.8 Hz, 1 H).

### Example 103

**2-(1-Benzyl-6-methoxy-1H-indol-2-yl)propan-2-ol (Compound 103).** To a solution of methyl 1-benzyl-6-methoxy-1H-indole-2-carboxylate (**Compound 102**, 4.33 g, 14.7 mmol) in THF (50 ml) at 0 °C under argon was added MeLi (3.0 M in diethoxymethane, 19.6 ml, 58.7 mmol) slowly. After 1 h, the ice-water bath was removed and the reaction was stirred at room temperature for 1h, cooled to -78 °C, quenched with dry ice, diluted with EtOAc, washed with H₂O, brine, dried over Na₂SO₄, concentrated *in vacuo* to yield the crude title compound as a yellow solid. ¹H NMR (500 MHz, CHLOROFORM-*d*) δ ppm 1.69 (s, 6 H), 3.73 (s, 3 H), 5.76 (s, 2 H), 6.42 (s, 1 H), 6.55 (d, *J* = 2.4 Hz, 1 H), 6.75 - 6.81 (m, 1 H), 6.96 (d, *J* = 7.3 Hz, 2 H), 7.22 (d, *J* = 7.3 Hz, 1 H), 7.25 - 7.30 (m, 2 H), 7.49 (d, *J* = 8.8 Hz, 1 H).

### Example 104

**1-Benzyl-2-isopropyl-6-methoxy-1H-indole (Compound 104).** To a solution of 2-(1-benzyl-6-methoxy-1H-indol-2-yl)propan-2-ol (**Compound 103**, 1.05 g, 3.57 mmol) in EtOAc (35 ml) and EtOH (15 ml) was added 10% Pd-C (190 mg, 0.18 mmol) and HCl-Et₂O (1.0 M, 1.25 ml, 1.25 mmol). The mixture was stirred under hydrogen gas (atmospheric pressure) for 1h and was filtered. To the filtrate was added NaHCO₃ (0.5 g) and H₂O (0.5 ml), followed by Na₂SO₄ and MgSO₄. This was then filtered and concentrated *in vacuo* to yield the crude title compound as a yellow solid.
¹H NMR (300 MHz, CHLOROFORM-*d*) δ ppm 1.31 (d, *J* = 6.7 Hz, 6 H), 2.90 - 3.10 (m, 1 H), 3.79 (s, 3 H), 5.33 (s, 2 H), 6.33 (s, 1 H), 6.68 (d, *J* = 2.1 Hz, 1 H), 6.79 (dd, *J* = 8.5, 2.3 Hz, 1 H), 6.94 - 7.04 (m, 2 H), 7.20 - 7.37 (m, 2 H), 7.49 (d, *J* = 8.5 Hz, 1 H).

### Example 105

**1-Benzyl-2-isopropyl-6-methoxy-1H-indole-3-carbaldehyde (Compound 105).** POCl₃ (0.48 ml, 5.23 mmol) was added dropwise to anhydrous DMF (2 ml) at 0 °C under argon. After stirred for 30 min, this solution was added dropwise to a solution of 1-benzyl-2-isopropyl-6-methoxy-1H-indole (**Compound 104**, 583 mg, 2.09 mmol) in anhydrous DMF (8 ml) at 0 °C under argon. The reaction was stirred for 1 h at 0 °C and 30 min at room temperature, diluted with EtOAc, washed with aqueous NaHCO₃, brine, dried over Na₂SO₄, and concentrated *in vacuo.* The residue was purified by chromatography on silica gel (0→30% EtOAc-hexanes) to yield the title compound as a light yellow syrup.
¹H NMR (500 MHz, CHLOROFORM-*d*) δ ppm 1.45 (d, *J* = 7.3 Hz, 6 H), 3.40 - 3.52 (m, 1 H), 3.79 (s, 3 H), 5.40 (s, 2 H), 6.69 (d, *J* = 2.4 Hz, 1 H), 6.94 (dd, *J* = 8.8, 2.0 Hz, 1 H), 7.01 (d, *J* = 7.3 Hz, 2 H), 7.25 - 7.35 (m, 3 H), 8.28 (d, *J* = 8.8 Hz, 1 H), 10.45 (s, 1 H).

### Example 106

**1-Benzyl-2-isopropyl-6-methoxy-1H-indole-3-carboxylic Acid (Compound 106).** To a solution of 1-benzyl-2-isopropyl-6-methoxy-1H-indole-3-carbaldehyde (**Compound 105**, 608 mg, 1.98 mmol) in t-BuOH (15 ml), CH₃CN (15 ml), and 2-methyl-2-butene (10 ml) was added a solution of KH₂PO₄ (5.4 g, 39.6 mmol) and NaClO₂ (80%, 4.5 g, 39.6 mmol) in H₂O (50 ml). The mixture was stirred at room temperature and additional 2-methyl-2-butene, KH₂PO₄, and NaClO₂ were added at the above ratio every 16-24 h until the starting material was consumed. The reaction mixture was extracted with EtOAc (×3) and the combined organic layer was washed with brine, dried over Na₂SO₄, and concentrated *in vacuo*. The residue was purified by chromatography on silica gel (0→25% EtOAc-hexanes) to yield the title compound as a yellow solid.
¹H NMR (500 MHz, CHLOROFORM-*d*) δ ppm 1.39 (d, *J* = 7.3 Hz, 6 H), 3.75 (s, 3 H), 3.99 - 4.17 (m, 1 H), 5.45 (s, 2 H), 6.62 (d, *J* = 2.4 Hz, 1 H), 6.90 (dd, *J* = 8.8, 2.4 Hz, 1 H), 6.99 (d, *J* = 7.3 Hz, 2 H), 7.22 - 7.34 (m, 3 H), 8.18 (d, *J* = 8.8 Hz, 1 H).

### Example 107

**1-Benzyl-N-(3,4-difluorobenzyl)-2-isopropyl-6-methoxy-1H-indole-3-carboxamide (Compound 107).** To a solution of 1-benzyl-2-isopropyl-6-methoxy-1H-indole-3-carboxylic acid (**Compound 106**, 226 mg, 0.70 mmol) in CH₂Cl₂ (7.0 ml) was added EDC (202 mg, 1.05 mmol) and DMAP (128 mg, 1.05 mmol) followed by 3,4-difluorobenzylamine (0.25 ml, 2.1 mmol). The reaction was stirred at room temperature for 18 h, diluted with EtOAc, washed with H₂O, brine, dried over Na₂SO₄ and concentrated *in vacuo.* The residue was purified by chromatography on silica gel (0→30% EtOAc-hexanes) to yield the title compound as a yellow solid.
¹H NMR (500 MHz, CHLOROFORM-*d*) δ ppm 1.37 (d, *J* = 7.3 Hz, 6 H), 3.65 - 3.73 (m, 1 H), 3.74 (s, 3 H), 4.66 (d, *J* = 5.9 Hz, 2 H), 5.40 (s, 2 H), 6.30 (t, *J* = 6.3 Hz, 1 H), 6.63 (d, *J* = 2.0 Hz, 1 H), 6.82 (dd, *J* = 8.8, 2.4 Hz, 1 H), 6.96 (d, *J* = 6.8 Hz, 2 H), 7.11 - 7.17 (m, 2 H), 7.21 - 7.31 (m, 4 H), 7.51 (d, *J* = 8.3 Hz, 1 H).

### Example 108

**1-Benzyl-N-(3,4-difluorobenzyl)-6-hydroxy-2-isopropyl-1H-indole-3-carboxamide (Compound 108).** To a solution of 1-benzyl-N-(3,4-difluorobenzyl)-2-isopropyl-6-methoxy-1H-indole-3-carboxamide (**Compound 107**, 452 mg, 1.0 mmol) in CH₂Cl₂ (20 ml) at 0 °C was added BBr₃ (1.0 M in CH₂Cl₂, 3.0 ml, 3.0 mmol) dropwise. The reaction was stirred for 1 h at 0 °C and 1 h at room temperature, quenched with ice, extracted with EtOAc, the organic layer was washed with brine, dried over Na₂SO₄, and concentrated *in vacuo.* The residue was purified by chromatography on silica gel (0→50% EtOAc-hexanes) to yield the title compound as a yellow solid.
¹H NMR (500 MHz, CHLOROFORM-*d*) δ ppm 1.37 (d, *J* = 7.3 Hz, 6 H), 3.65 - 3.74 (m, 1 H), 4.66 (d, *J* = 5.9 Hz, 2 H), 4.78 (s, 1 H), 5.37 (s, 2 H), 6.27 (t, *J* = 5.6 Hz, 1 H), 6.60 (d, *J* = 2.4 Hz, 1 H), 6.71 (dd, *J* = 8.5, 2.2 Hz, 1 H), 6.95 (d, *J* = 6.8 Hz, 2 H), 7.11 - 7.17 (m, 2 H), 7.21 - 7.32 (m, 4 H), 7.46 (d, *J* = 8.8 Hz, 1 H).

### Example 109

**1-benzyl-N-(3,4-difluorobenzyl)6-ethoxy-2-isopropyl-1H-indole-3-carboxamide (Compound 109). General Procedure A.** To a solution of 1-benzyl-N-(3,4-difluorobenzyl)-6-hydroxy-2-isopropyl-1H-indole-3-carboxamide (**Compound 108**, 40 mg, 0.092 mmol) in DMF (2.0 ml) was added K₂CO₃ (39 mg, 0.28 mmol) and iodoethane (22 µl, 0.28 mmol). The reaction was stirred at room temperature for 48 h, diluted with EtOAc, washed with H₂O, brine, dried over Na₂SO₄ and concentrated *in vacuo.* The residue was purified by PTLC on silica gel (30% EtOAc-hexanes) to yield the title compound as an off-white solid.
¹H NMR (500 MHz, CHLOROFORM-*d*) δ ppm 1.37 (t, *J* = 7.0 Hz, 3 H), 1.38 (d, *J* = 7.3 Hz, 6 H), 3.68 - 3.75 (m, 1 H), 3.96 (q, *J* = 7.0 Hz, 2 H), 4.67 (d, *J* = 6.3 Hz, 2 H), 5.40 (s, 2 H), 6.31 (t, *J* = 5.4 Hz, 1 H), 6.64 (d, *J* = 2.4 Hz, 1 H), 6.82 (dd, *J* = 8.8, 2.0 Hz, 1 H), 6.97 (d, *J* = 6.8 Hz, 2 H), 7.13 - 7.17 (m, 2 H), 7.23 - 7.31 (m, 4 H), 7.52 (d, *J* = 8.3 Hz, 1 H)

### Example 110

**1-Benzyl-N-(3,4-difluorobenzyl)-2-isopropyl-6-propoxy-1H-indole-3-carboxamide (Compound 110).** Following General Procedure A, 1-benzyl-N-(3,4-difluorobenzyl)-6-hydroxy-2-isopropyl-1H-indole-3-carboxamide (**Compound 108**, 8.0 mg, 0.018 mmol) in DMF (1.0 ml) was reacted with K₂CO₃ (8.0 mg, 0.055 mmol) and 1-iodopropane (9.0 µl, 0.092 mmol) to yield the title compound as a white solid.
¹H NMR (500 MHz, METHANOL-*d*₄) δ ppm 0.99 (t, *J* = 7.6 Hz, 3 H), 1.32 (d, *J* = 7.3 Hz, 6 H), 1.67 - 1.77 (m, 2 H), 3.42 - 3.53 (m, 1 H), 3.84 (t, *J* = 6.6 Hz, 2 H), 4.57 (s, 2 H), 5.46 (s, 2 H); 6.73 (d, *J* = 2.0 Hz, 1 H), 6.78 (dd, *J* = 8.8, 2.4 Hz, 1 H), 6.95 (d, *J* = 6.8 Hz, 2 H), 7.19 - 7.29 (m, 5 H), 7.30 - 7.36 (m, 1 H), 7.49 (d, *J* = 8.3 Hz, 1 H).

### Example 111

**1-Benzyl-N-(3,4-difluorobenzyl)-6-isopropoxy-2-isopropyl-1H-indole-3-carboxamide (Compound 111).** Following General Procedure A, 1-benzyl-N-(3,4-difluorobenzyl)-6-hydroxy-2-isopropyl-1H-indole-3-carboxamide (**Compound 108**, 8.0 mg, 0.018 mmol) in DMF (1.0 ml) was reacted with K₂CO₃ (8.0 mg, 0.055 mmol) and 2-iodopropane (9.0 µl, 0.092 mmol) to yield the title compound as a white solid.
¹H NMR (500 MHz, METHANOL-*d*₄) δ ppm 1.21 (d, *J* = 5.9 Hz, 6 H), 1.33 (d, *J* = 7.3 Hz, 6 H), 3.45 - 3.55 (m, 1 H), 4.41 - 4.50 (m, 1 H), 4.57 (s, 2 H), 5.46 (s, 2 H), 6.72 (d, *J* = 2.0 Hz, 1 H), 6.74 - 6.79 (m, 1 H), 6.96 (d, *J* = 7.3 Hz, 2 H), 7.18 - 7.29 (m, 5 H), 7.30 - 7.37 (m, 1 H), 7.49 (d, *J* = 8.8 Hz, 1 H).

### Example 112

**1-Benzyl-6-butoxy-N-(3,4-difluorobenzyl)-2-isopropyl-1H-indole-3-carboxamide (Compound 112).** Following General Procedure A, 1-benzyl-N-(3,4-difluorobenzyl)-6-hydroxy-2-isopropyl-1H-indole-3-carboxamide (**Compound 108**, 10.7 mg, 0.025 mmol) in DMF (1.0 ml) was reacted with K₂CO₃ (10.0 mg, 0.074 mmol) and 1-iodobutane (14.0 µl, 0.12 mmol) to yield the title compound as a white solid.
¹H NMR (500 MHz, CHLOROFORM-*d*) δ ppm 0.93 (t, *J* = 7.3 Hz, 3 H), 1.37 (d, *J* = 7.3 Hz, 6 H), 1.40 - 1.50 (m, 2 H), 1.66 - 1.74 (m, 2 H), 3.61 - 3.75 (m, 1 H), 3.88 (t, *J* = 6.6 Hz, 2 H), 4.66 (d, *J* = 6.3 Hz, 2 H), 5.39 (s, 2 H), 6.30 (t, *J* = 5.9 Hz, 1 H), 6.63 (d, *J* = 2.0 Hz, 1 H); 6.81 (dd, *J* = 8.5, 2.2 Hz, 1 H), 6.96 (d, *J* = 6.8 Hz, 2 H), 7.10 - 7.17 (m, 2 H), 7.21 - 7.32 (m, 4 H), 7.50 (d, *J* = 8.8 Hz, 1 H).

### Example 113

**1-Benzyl-N-(3,4-difluorobenzyl)-6-isobutoxy-2-isopropyl-1H-indole-3-carboxamide (Compound 113).** Following General Procedure A, 1-benzyl-N-(3,4-difluorobenzyl)-6-hydroxy-2-isopropyl-1H-indole-3-carboxamide (**Compound 108**, 10.7 mg, 0.025 mmol) in DMF (1.0 ml) was reacted with K₂CO₃ (10.0 mg, 0.074 mmol) and 2-iodobutane (14.0 µl, 0.12 mmol) to yield the title compound as a white solid.
¹H NMR (500 MHz, CHLOROFORM-*d*) δ ppm 0.98 (d, *J* = 6.8 Hz, 6 H), 1.36 (d, *J* = 7.3 Hz, 6 H), 1.96 - 2.08 (m, 1 H), 3.65 (d, *J* = 6.8 Hz, 2 H), 3.65 - 3.72 (m, 1 H), 4.66 (d. *J* = 6.3 Hz, 2 H), 5.39 (s, 2 H), 6.29 (t, *J* = 5.6 Hz, 1 H), 6.63 (d, *J* = 2.0 Hz, I H), 6.82 (dd, *J* = 8.8, 2.0 Hz, 1 H), 6.96 (d, *J* = 6.8 Hz, 2 H), 7.11 - 7.16 (m, 2 H), 7.21 - 7.31 (m, 4 H), 7.50 (d, *J* = 8.8 Hz, 1 H).

### Example 114

**1-Benzyl-N-(3,4-difluorobenzyl)-6-(hexoxy)-2-isopropyl-1H-indole-3-carboxamide (Compound 114).** Following General Procedure A, 1-benzyl-N-(3,4-difluorobenzyl)-6-hydroxy-2-isopropyl-1H-indole-3-carboxamide (**Compound 108**, 10.7 mg, 0.025 mmol) in DMF (1.0 ml) was reacted with K₂CO₃ (10.0 mg, 0.074 mmol) and 1-iodohexane (18.0 µl, 0.12 mmol) to yield the title compound as a white solid.
¹H NMR (500 MHz, CHLOROFORM-*d*) δ ppm 0.85 - 0.93 (m, 3 H), 1.24 - 1.33 (m, 4 H), 1.37 (d, *J* = 6.8 Hz, 6 H), 1.38 - 1.46 (m, 2 H), 1.66 - 1.77 (m, 2 H), 3.63 - 3.75 (m, 1 H), 3.87 (t, *J* = 6.6 Hz, 2 H), 4.66 (d, *J* = 5.9 Hz, 2 H), 5.39 (s, 2 H), 6.30 (t, *J* = 5.6 Hz, 1 H), 6.63 (d, *J* = 2.4 Hz, 1 H), 6.81 (dd, *J* = 8.8, 2.4 Hz, 1 H), 6.96 (d, *J* = 6.8 Hz, 2 H), 7.10 - 7.16 (m, 2 H), 7.21 - 7.31 (m, 4 H), 7.50 (d, *J* = 8.8 Hz, 1 H).

### Example 115

**1-Benzyl-6-(benzyloxy)-N-(3,4-difluorobenzyl)-2-isopropyl-1H-indole-3-carboxamide (Compound 115).** Following General Procedure A, 1-benzyl-N-(3,4-difluorobenzyl)-6-hydroxy-2-isopropyl-1H-indole-3-carboxamide (**Compound 108**, 10.7 mg, 0.025 mmol) in DMF (1.0 ml) and acetone (1.0 ml) was reacted with K₂CO₃ (10.0 mg, 0.074 mmol), benzyl bromide (14.0 µl, 0.12 mmol), and catalytic amount of NaI to yield the title compound as an off-white solid.
¹H NMR (500 MHz, CHLOROFORM-*d*) δ ppm 1.37 (d, *J* = 7.3 Hz, 6 H), 3.65 - 3.75 (m, 1 H), 4.66 (d, *J* = 6.3 Hz, 2 H), 4.99 (s, 2 H), 5.37 (s, 2 H), 6.28 (t, *J* = 6.3 Hz, 1 H), 6.71 (d, *J* = 2.0 Hz, 1 H), 6.89 (dd, *J* = 8.8, 2.0 Hz, 1 H), 6.95 (d, *J* = 6.8 Hz, 2 H), 7.11 - 7.18 (m, 2 H), 7.22 - 7.30 (m, 5 H), 7.31 - 7.39 (m, 4 H), 7.51 (d, *J* = 8.8 Hz, 1 H).

### Example 116

**1-Benzyl-6-(cyclopentoxy)-N-(3,4-difluorobenzyl)-2-isopropyl-1H-indole-3-carboxamide (Compound 116).** Following General Procedure A, 1-benzyl-N-(3,4-difluorobenzyl)-6-hydroxy-2-isopropyl-1H-indole-3-carboxamide (**Compound 108**, 40 mg, 0.092 mmol) in DMF (1.0 ml) was reacted with K₂CO₃ (38 mg, 0.28 mmol), cyclopentyl iodide (53 µl, 0.46 mmol) to yield the title compound as a white solid.
¹H NMR (300 MHz, CHLOROFORM-*d*) δ ppm 1.37 (d, *J* = 7.0 Hz, 6 H), 1.48 - 1.60 (m, 2 H), 1.66 - 1.86 (m, 6 H), 3.62 - 3.83 (m, 1 H), 4.56 - 4.77 (m, 3 H), 5.38 (s, 2 H), 6.32 (t, *J* = 5.9 Hz, 1 H), 6.61 (d, *J* = 2.1 Hz, 1 H), 6.78 (dd, *J* = 8.8, 2.1 Hz, 1 H), 6.91 - 7.02 (m, 2 H), 7.08 - 7.17 (m, 2 H), 7.17 - 7.36 (m, 4 H), 7.49 (d, *J* = 8.5 Hz, 1 H).

### Example 117

**1-Benzyl-N-(3,4-difluorobenzyl)-2-isopropyl-6-(2-methoxyethoxy)-1H-indole-3-carboxamide (Compound 117).** Following General Procedure A, 1-benzyl-N-(3,4-difluorobenzyl)-6-hydroxy-2-isopropyl-1H-indole-3-carboxamide (**Compound 108, 17** mg, 0.039 mmol) in DMF (1.0 ml) was reacted with K₂CO₃ (28 mg, 0.20 mmol), 2-bromoethyl methyl ether (18 µl, 0.20 mmol) to yield the title compound (9 mg, 49%).
¹H NMR (300 MHz, CDCl₃) δ ppm 1.37 (d, *J* = 7.04 Hz, 6 H), 3.40 (s, 3 H), 3.60 - 3.78 (m, 3 H), 4.04 (dd, *J* = 5.42, 3.96 Hz, 2 H), 4.66 (d, *J* = 5.86 Hz, 2 H), 5.39 (s, 2 H), 6.30 (t, *J* = 5.86 Hz, 1 H), 6.68 (d, *J* = 2.35 Hz, 1 H), 6.85 (dd, *J* = 8.65, 2.20 Hz, 1 H), 6.89 - 7.01 (m, 2 H), 7.10 - 7.18 (m, 2 H), 7.17 - 7.35 (m, 4 H), 7.51 (d, J = 8.79 Hz, 1 H).

### Example 118

**1-Benzyl-N-(3,4-difluorobenzyl)-6-(2-(dimethylamino)ethoxy)-2-isopropyl-1H-indole-3-carboxamide (Compound 118)**. Following General Procedure A, 1-benzyl-N-(3,4-difluorobenzyl)-6-hydroxy-2-isopropyl-1H-indole-3-carboxamide (**Compound 108**, 17 mg, 0.039 mmol) in DMF (1.0 ml) was reacted with K₂CO₃ (28 mg, 0.20 mmol), 2-dimehtylamino ethyl chloride hydrochloride (20 mg, 0.20 mmol) to yield the title compound (10 mg, 53%).
¹H NMR (300 MHz, CD₃OD) δ ppm 1.32 (d, *J* = 7.04 Hz, 6 H), 2.30 (s, 6 H), 2.71 (t, *J* = 5.42 Hz, 2 H), 3.37 - 3.59 (m, 1 H), 4.02 (t, *J* = 5.42 Hz, 2 H), 4.57 (s, 2 H), 5.48 (s, 2 H), 6.73 - 6.88 (m, 2 H), 6.89 - 7.02 (m, 2 H), 7.12 - 7.40 (m, 6 H), 7.50 (d, *J* = 8.50 Hz, 1 H).

### Example 119

**1-Benzyl-N-(3,4-difluorobenzyl)-2-isopropyl-6-(tetrahydrofuran-3-yloxy)-1H-indole-3-carboxamide (Compound 119)**. To a solution of 1-benzyl-N-(3,4-difluorobenzyl)-6-hydroxy-2-isopropyl-1H-indole-3-carboxamide (**Compound 108,** 8 mg, 0.039 mmol) in DMF (1.0 ml) was added K₂CO₃ (13 mg, 0.092 mmol) and catalytic amount of NaOH, 3-iodotetrahydrofuran (**Compound 29,** 120 mg, crude). The reaction was stirred at room temperature for 2 days, and purified by a short silica gel column to yield the title compound (8 mg, 86%).
¹H NMR (300 MHz, CDCl₃) δ ppm 1.38 (d, *J* = 7.04 Hz, 6 H), 1.95 - 2.14 (m, 2 H), 3.59 - 4.01 (m, 5 H), 4.66 (d, *J* = 6.16 Hz, 2 H), 4.74 - 4.88 (m, 1 H), 5.39 (s, 2 H), 6.29 (t, *J* = 4.40 Hz, 1 H), 6.57 (d, *J* = 2.05 Hz, 1 H), 6.69 - 6.83 (m, 1 H), 6.96 (d, *J* = 7.62 Hz, 2 H), 7.08 - 7.19 (m, 2 H), 7.18 - 7.35 (m, 4 H), 7.51 (d, *J* = 8.79 Hz, 1 H).

### Example 120

**1-Benzyl-N-(3,4-difluorobenzyl)-2-isopropyl-6-(2-oxotetrahydrofuran-3-yloxy)-1H-indole-3-carboxamide (Compound 120)**. Following General Procedure A, 1-benzyl-N-(3,4-difluorobenzyl)-6-hydroxy-2-isopropyl-1H-indole-3-carboxamide (**Compound 108**, 19mg, 0.044mol) in DMF (1.0 ml) was reacted with K₂CO₃ (30 g, 0.22 mmol), 3-bromodihydrofuran-2(3H)-one (20 mg, 0.22mmol) to yield the title compound (16mg, 71 %).
¹H NMR (300 MHz, acetone-d₆) δ ppm 1.33 (d, *J* = 5.57 Hz, 6 H), 2.21 - 2.42 (m, 1 H), 2.68 - 2.88 (m, 1 H), 3.43 - 3.65 (m, 1 H), 4.21 - 4.53 (m, 2 H), 4.66 (d, *J* = 6.16 Hz, 2 H), 5.10 - 5.24 (m, 1 H), 5.54 (s, 2 H), 6.90 (dd, *J* = 8.65, 2.20 Hz, 1 H), 6.97 - 7.08 (m, 2 H), 7.11 (d, *J* = 2.35 Hz, 1 H), 7.17 - 7.35 (m, 5 H), 7.42 (dd, *J* = 12.31, 8.50 Hz, 1 H), 7.62 (d, *J* = 8.79 Hz, 1 H), 7.68 - 7.78 (m, 1 H).

### Example 121

### 1-benzyl-3-(3,4-difluorobenzylcarbamoyl)-2-isopropyl-1H-indol-6-yl

**Dimethylcarbamate (Compound 121). General Procedure B**. To a solution of 1-benzyl-N-(3,4-difluorobenzyl)-6-hydroxy-2-isopropyl-1H-indole-3-carboxamide (**Compound 108**, 18mg, 0.041mol) in pyridine (1 ml) was added dimethylcarbamyl chloride (40 µl, 0.41 mmol) and stirred at room temperature overnight. The reaction was quenched with water, extracted with ethyl acetate. The combined organic layer was washed with water, brine, dried over Na₂SO₄, and concentrated *in vacuo.* The residue was purified by chromatography on silica gel (0→50% EtOAc-hexanes) to yield the title compound as a white solid (17 mg, 82%).
¹H NMR (300 MHz, CD₃OD) δ ppm 1.32 (d, *J* = 7.04 Hz, 6 H), 2.96 (s, 3 H), 3.09 (s, 3 H), 3.37 - 3.55 (m, 1 H), 4.58 (s, 2 H), 5.48 (s, 2 H), 6.87 (dd, *J* = 8.65, 1.91 Hz, 1 H), 6.91 - 6.99 (m, 2 H),7.02 (d, *J* = 2.05 Hz, 1 H), 7.16 - 7.39 (m, 6 H), 7.58 (d, *J* = 8.79 Hz, 1 H).

### Example 122

**1-Benzyl-3-(3,4-difluorobenzylcarbamoyl)-2-isopropyl-1H-indol-6-yl Pivalate (Compound 122)**. Following General Procedure B, 1-benzyl-N-(3,4-difluorobenzyl)-6-hydroxy-2-isopropyl-1H-indole-3-carboxamide (**Compound 108**, 18mg, 0.041mol) in pyridine (1 ml) was reacted with pivaloyl chloride (5.1 µl, 0.41 mmol) to yield the title compound (16 mg, 74%).
¹H NMR (300 MHz, CD₃OD) δ ppm 1.25 - 1.40 (m, 15 H), 3.34 - 3.55 (m, 1 H), 4.58 (d, *J* = 5.86 Hz, 2 H), 5.49 (s, 2 H), 6.73 - 6.88 (m, 1 H), 6.89 - 6.99 (m, 2 H), 7.00 (d, *J* = 1.76 Hz, 1 H), 7.14 - 7.41 (m, 6 H), 7.60 (d, *J* = 8.50 Hz, 1 H).

### Example 123

**1-Benzyl-3-(3,4-difluorobenzylcarbamoyl)-2-isopropyl-1H-indol-6-yl Acetate (Compound 123)**. Following General Procedure B, 1-benzyl-N-(3,4-difluorobenzyl)-6-hydroxy-2-isopropyl-1H-indole-3-carboxamide **(Compound 108**, 7mg, 0.016mol) in pyridine (1 ml) was reacted with acetyl chloride (1.0 µl, 0.16 mmol) to yield the title compound (8 mg, 100%).
¹H NMR (300 MHz, CDCl₃) δ ppm 1.37 (d, *J* = 7.04 Hz, 6 H), 2.26 (s, 3 H), 3.54 - 3.76 (m, 1 H), 4.66 (d, *J* = 6.16 Hz, 2 H), 5.41 (s, 2 H), 6.28 (t, *J* = 6.01 Hz, 1 H), 6.81 - 7.01 (m, 4 H), 7.06 - 7.19 (m, 2 H), 7.18 - 7.35 (m, 4 H), 7.61 (d, *J* = 9.09 Hz, 1 H).

### Example 124

**1-Benzyl-3-(3,4-difluorobenzylcarbamoyl)-2-isopropyl-1H-indol-6-yl Propionate (Compound 124)**. Following General Procedure B, 1-benzyl-N-(3,4-difluorobenzyl)-6-hydroxy-2-isopropyl-1H-indole-3-carboxamide **(Compound 108**, 7mg, 0.016mol) in pyridine (1 ml) was reacted with propionyl chloride (1.4 µl, 0.16 mmol) to yield the title compound (8 mg, 100%).
¹H NMR (300 MHz, EDCl₃) δ ppm 1.23 (t, *J* = 7.48 Hz, 3 H), 1.37 (d, *J* = 7.33 Hz, 6 H), 2.55 (q, *J* = 7.43 Hz, 2 H), 3.53 - 3.73 (m, 1 H), 4.66 (d, *J* = 5.86 Hz, 2 H), 5.41 (s, 2 H), 6.30 (t, *J* = 5.72 Hz, 1 H), 6.83 - 7.00 (m, 4 H), 7.06 - 7.18 (m, 2 H), 7.18 - 7.35 (m, 4 H), 7.60 (d, *J* = 8.50 Hz, 1 H).

### Example 125

**1-Benzyl-3-(3,4-difluorobenzylcarbamoyl)-2-isopropyl-1H-indol-6-yl Isobutyrate (Compound 125)**. Following General Procedure B, 1-benzyl-N-(3,4-difluorobenzyl)-6-hydroxy-2-isopropyl-1H-indole-3-carboxamide **(Compound 108**, 9mg, 0.021mol) in pyridine (1 ml) was reacted with isobutyryl chloride (4.1 µl, 0.21 mmol) to yield the title compound (8 mg, 80%).
¹H NMR (300 MHz, CDCl₃) δ ppm 1.13 - 1.42 (m, 12 H), 2.47 - 2.85 (m, 1 H), 3.50 - 3.74 (m, 1 H), 4.66 (d, *J* = 6.16 Hz, 2 H), 5.41 (s, 2 H), 6.20 - 6.44 (m, 1 H), 6.74 - 7.00 (m, 4 H), 7.07 - 7.18 (m, 2 H), 7.17 - 7.35 (m, 4 H), 7.60 (d, *J* = 8.50 Hz, 1 H).

### Example 126

**1-Benzyl-N-(3,4-difluorobenzyl)-2-isopropyl-6-(methoxymethoxy)-1H-indole-3-carboxamide (Compound 126)**. To a solution of 1-benzyl-N-(3,4-difluorobenzyl)-6-hydroxy-2-isopropyl-1H-indole-3-carboxamide **(Compound 108**, 39 mg, 0.090 mmol) in CH₂Cl₂ (2.0 ml) was added *i*-Pr₂NEt (47 µl, 0.27 mmol) and MOMCl (35 µl, 0.45 mmol). The reaction was stirred at room temperature for 4 h, and was purified directly by PTLC on silica gel (30% EtOAc-hexanes) to yield the title compound as a white solid.
¹H NMR (300 MHz, CHLOROFORM-*d*) δ ppm 1.37 (d, *J* = 7.0 Hz, 6 H), 3.42 (s, 3 H), 3.59 - 3.78 (m, 1 H), 4.66 (d, *J* = 5.9 Hz, 2 H), 5.10 (s, 2 H), 5.40 (s, 2 H), 6.29 (t, *J* = 5.7 Hz, 1 H), 6.85 (d, *J* = 2.1 Hz, 1 H), 6.89 - 7.01 (m, 3 H), 7.10 - 7.17 (m, 2 H), 7.20 - 7.34 (m, 4 H), 7.52 (d, *J* = 8.5 Hz, 1 H).

### Example 127

**1-Benzyl-N-(3,4-difluorobenzyl)-2-isopropyl-6-(tetrahydrofuran-2-yloxy)-1H-indole-3-carboxamide (Compound 127)**. To a solution of 1-benzyl-N-(3,4-difluorobenzyl)-6-hydroxy-2-isopropyl-1H-indole-3-carboxamide **(Compound 108**, 39 mg, 0.090 mmol) in CH₂Cl₂ (2.0 ml) was added 2,3-dihydrofuran (68 µl, 0.90 mmol) and catalytic amount of PPTS. The reaction was stirred at room temperature for 4 h, and was purified directly by PTLC on silica gel (30% EtOAc-hexanes) to yield the title compound as a white solid.
¹H NMR (300 MHz, CHLOROFORM-*d*) δ ppm 1.36 (d, *J* = 7.3 Hz, 6 H), 1.85 - 1.98 (m, 1 H), 2.01 - 2.19 (m, 3 H), 3.58 - 3.73 (m, 1 H), 3.85 - 3.95 (m, 1 H), 3.96 - 4.07 (m, 1 H), 4.66 (d, *J* = 5.9 Hz, 2 H), 5.40 (s, 2 H), 5.70 (d, *J* = 4.7 Hz, 1 H), 6.29 (t, *J* = 5.7 Hz, 1 H), 6.86 (d, *J* = 2.1 Hz, 1 H), 6.89 - 6.99 (m, 3 H), 7.11 - 7.17 (m, 2 H), 7.19 - 7.32 (m, 4 H), 7.51 (d, *J* = 8.5 Hz, 1 H).

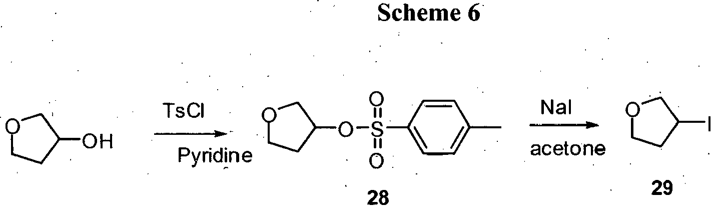

### Example 128

**Tetrahydrofuran-3-yl 4-methylbenzenesulfonate (Compound 128 or Compound 29, Scheme 6)**. To a solution of tetrahydrofuran-3-ol (500 mg, 5.67 mmol) in pyridine (10 ml) at 0 °C was added 4-methylbenzene-1-sulfonyl chloride (1.08 g, 5.67 mmol). The reaction was stirred at room temperature overnight. The reaction was quenched with water, extracted with ethyl acetate. The organic layer was washed with water, brine, dried over Na₂SO₄ and concentrated in vacuo to yield crude oil (1.2 g).
¹H NMR (300 MHz, CDCl₃) δ ppm 1.91 - 2.23 (m, 2 H), 3.61 - 4.05 (m, 4 H), 4.95 - 5.24 (m, 1 H), 7.36 (d, *J* = 7.92 Hz, 2 H), 7.80 (d, *J* = 8.50 Hz, 2 H).

### Example 129

**3-Iodotetrahydrofuran (Compound 129)**. To a solution of crude tetrahydrofuran-3-yl 4-methylbenzenesulfonate (**Compound 128**, 1.2 g, 4.96 mmol) in dry acetone (50 ml) was added NaI (1.1 g, 7.44 mmol). The reacted was heated at 60 °C for 2 days. The mixture was diluted with water and extracted with diethyl ether. The organic layer was washed with water, brine, dried over Na₂SO₄ and concentrated in vacuo to yield crude oil which was used directly without purification.
¹H NMR (300 MHz, CDCl₃) δ ppm 2.23 - 2.55 (m, 2 H), 3.81 - 4.08 (m, 3 H), 4.08 - 4.43 (m, 2H).

In particular, the compounds of the invention comprise a 6-substituted indole-3-carboxylic acid-N- arylmethyl amide having spingosine-1-phosphate antagonist activity wherein the 6-substituent is represented by the formula (X¹)ᵣ-A²-B
wherein X¹ is O;
r is 0 or 1;
A² is absent or is (CH₂)ᵥ, wherein v is 1 or 2;
B is OR⁶ or NR⁸R⁹, wherein R⁶, R⁸ and R⁹ are methyl; or
B is CR¹⁰=NO R¹¹R¹⁰ wherein R¹⁰ is H and
R¹¹ is methyl or i-butyl; or
B is CONR⁸R⁹, wherein R⁸ and R⁹ are selected from the group consisting of H, methyl, ethyl and propyl or R⁸ and R⁹, together with N, form a 5-membered ring; or
B is OR⁶, wherein R⁶ is H; or
B is COR¹⁰, wherein R¹⁰ is methyl.

## Claims

1. Compounds represented by having sphingosine-1-phosphate receptor agonist and/or antagonist biological activity represented by the general formula: 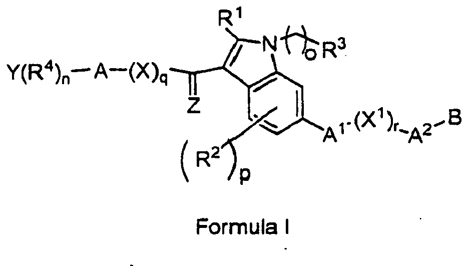 wherein:
R¹ R², R³ and R⁴ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, carbocyclic hydrocarbon groups having from 3 to 20 carbon atoms, heterocyclic groups having up to 20 carbon atoms and at least one of oxygen, nitrogen and/or sulfur in the ring, halo, C₁ to C₁₂ haloalkyl, hydroxyl, C₁ to C₁₂ alkoxy, C₃ to C₂₀ arylalkyloxy, C₁ to C₁₂ alkylcarbonyl, formyl, oxycarbonyl, carboxy, C₁ to C₁₂ alkyl carboxylate, C₁ to C₁₂ alkyl amide, aminocarbonyl, amino, cyano, diazo, nitro, thio, sulfoxyl, and sulfonyl groups;
X is NH;
X¹ is selected from the group consisting of NR⁵, O and S;
R⁵ is hydrogen, an alkyl group of I to 10 carbons, a cycloalkyl group of 5 to 10 carbons, phenyl or alkylphenyl wherein the alkyl group has 1-7 carbon atoms;
Y is a phenyl group or a pyridyl group, each of which may be bonded to A at any position;
Z is O;
n is 0 or an integer of from 1 to 5;
o is 0 or an integer of from 1 to 3;
p is 0 or an integer of from 1 to 3;
q is 1;
r is 0 or 1;
A is CH₂;
A¹ and A² are independently selected from the group consisting of (CH₂)ᵥ
wherein v is 0 or an integer of from 1 to 12, branched chain alkyl having 3 to 12 carbons, cycloalkyl having 3 to 12 carbons, alkenyl having 2 to 10 carbons and 1-3 double bonds and alkynyl having 2 to 10 carbons and 1 to 3 triple bonds;
B is selected from the group consisting of hydrogen, OR⁶, COOR⁷, NR⁸R⁹, CONR⁸R⁹, COR¹⁰, CH=NOR¹¹, CH=NNR¹²R¹³ wherein R⁶, R⁷, R¹⁰ and R¹¹ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, a carbocyclic hydrocarbon group having from 3 to 20 carbon atoms, a heterocyclic group having up to 20 carbon atoms and at least one of oxygen, nitrogen and/or sulfur in the ring, R⁸, R⁹, R¹² and R¹³ are independently selected from the group consisting of hydrogen, straight or branched chain alkyl having 1 to 12 carbons, alkenyl having 2 to 6 carbons and 1 or 2 double bonds, alkynyl having 2 to 6 carbons and 1 or 2 triple bonds, a carbocyclic hydrocarbon group having from 3 to 20 carbon atoms, a heterocyclic group having up to 20 carbon atoms and at least one of oxygen, nitrogen and/or sulfur in the ring, or R⁸ and R⁹ and/or R¹² and R¹³, together, can form a divalent carbon radical of 2 to 5 carbons to form a heterocyclic ring with nitrogen, wherein any of R⁶, R⁷, R⁸, R⁹, R¹⁰, R¹¹, R¹² or R¹³ may be substituted with one or more halogen, hydroxy, alkyloxy, cyano, nitro, mercapto or thiol radical; provided however, when v is 0, and r is 0, B is not hydrogen; or B is a carbocyclic hydrocarbon group having from 3 to 20 carbon atoms, or a heterocyclic group having up to 20 carbon atoms and at least one of oxygen, nitrogen and/or sulfur in the ring, and wherein when said B is a carbocyclic or heterocyclic group B may be bonded to A² at any position, or a pharmaceutically acceptable salt of said compound.

2. The compound of claim 1 wherein n is 0 or an integer of 1 or 2 and R⁴ is fluoro.

3. The compound of claim 2 wherein R¹ is i-propyl.

4. The compound of claim 3 wherein R³ is selected from the group consisting of phenyl, which may be substituted with one or two fluoro groups, and pyridyl.

5. The compound of claim 4 wherein p is 0.

6. The compound of claim 5 wherein A¹ and A² are absent.

7. The compound of claim 6 wherein B is OR⁶ or COOR⁷.

8. The compound of claim 6 wherein X¹ is O, r is 1, A¹ is absent, A² is (CH₂)ᵥ, wherein v is 1 or 2, and B is OR⁶ or NR⁸R⁹.

9. The compound of claim 8 wherein R⁶, R⁸ and R⁹ are methyl.

10. The compound of claim 6 wherein B is CR¹⁰=NOR¹¹R¹⁰ wherein R¹⁰ is H and R¹¹ is methyl or i-butyl, or wherein B is CONR⁸R⁹ wherein R⁸ and R⁹ are selected from the group consisting of H, methyl, ethyl and propyl, or R⁸ and R⁹, together with N, form a 5-member ring.

11. The compound of claim 6 wherein A¹ is absent, r is 0, A² is CH₂ and B is OR⁶, wherein R⁶ is H.

12. A compound selected from the group consisting of
1-Benzyl-5-hydroxy-2-methyl-1H-indole-3-carboxylic Acid, 3,5-Difluorobenzylamide;
5-Hydroxy-2-methyl-1-thiophen-2-ylmethyl-1H-indole-3-carboxylic Acid, 3, 4-Difluorobenzylamide;
1-Butyl-5-hydroxy-2-methyl-1H-indole-3-carboxylic Acid, 3, 5-Difluorobenzylamide;
1-Furan-2-ylmethyl-5-hydroxy-2-methyl-1H-indole-3-carboxylic Acid, 3, 4-Difluorobenzylamide;
5-Hydroxy-2-methyl-1-thiophen-2-ylmethyl-1H-indole-3-carboxylic Acid, 3, 5-Difluorobenzylamide;
1-Furan-2-ylmethyl-5-hydroxy-2-methyl-1H-indole-3-carboxylic Acid 3, 5-Difluorobenzylamide;
1-Benzyl-5-hydroxy-2-methyl-1H-indole-3-carboxylic Acid. 3, 4-Difluorobenzylamide;
5-Hydroxy-2-methyl-1-thiophen-2-ylmethyl-1H-indole-3-carboxylic Acid, 3-Fluorobenzylamide;
5-Hydroxy-2-methyl-1-thiophen-2-ylmethyl-1H-indole-3-carboxylic Acid, Benzylamide;
5-Hydroxy-2-methyl-1-thiophen-2-ylmethyl-1H-indole-3-carboxylic Acid, 3-Methoxybenzylamide;
1-Butyl-5-hydroxy-2-methyl-1H-indole-3-carboxylic Acid, 3-Methoxy-benzylamide;
5-Hydroxy-2-methyl-1-thiophen-2-ylmethyl-1H-indole-3-carboxylic Acid, 4-Fluorobenzylamide;
5-Hydroxy-2-methyl-1-thiophen-2-ylmethyl-1H-indole-3-carboxylic Acid, 4-Methylbenzylamide;
5-Hydroxy-2-methyl-1-thiophen-2-ylmethyl-1H-indole-3-carboxylic Acid, 3-Chlorobenzylamide;
5-Hydroxy-2-methyl-1-thiophen-2-ylmethyl-1H-indole-3-carboxylic Acid, 4-Chlorobenzylamide;
5-Hydroxy-2-methyl-1-thiophen-2-ylmethyl-1H-indole-3-carboxylic Acid, 2-methoxybenzylamide;
1-Benzyl-2-ethyl-5-hydroxy-1H-indole-3-carboxylic Acid, 3,4-Difluorobenzylamide;
1-Benzyl-2-ethyl-5-hydroxy-1H-indole-3-carboxylic Acid, 3-Methoxy-benzylamide;
1-Benzyl-5-hydroxy-2-isopropyl-1H-indole-3-carboxylic Acid, 3,4-Difluorobenzamide;
5-Hydroxy-2-methyl-1-phenyl-1H-indole-3-carboxylic Acid 3,4-Difluorobenzylamide;
5-Hydroxy-2-methyl-1-pyridin-2-yl-1H-indole-3-carboxylic Acid 3,4-Difluoro-benzylamide;
5-Hydroxy-2-methyl-1-thiophen-2-yl-1H-indole-3-carboxylic Acid 3,4-Difluorobenzylamide;
1-Benzyl-2-ethyl-5-hydroxy-1H-indole-3-carboxylic Acid 3,5-Difluoro-benzylamide;
1-Benzyl-5-hydroxy-2-isopropyl-1H-indole-3-carboxylic Acid, 3,5-difluorobenzylamide;
1-Benzyl-5-hydroxy-2-isopropyl-1H-indole-3-carboxylic Acid, 3-methoxybenzylamide; and
1-Benzyl-5-hydroxy-2-phenyl-1H-indole-3-carboxylic Acid, 3,5-Difluorobenzylamide.

13. The compound of claim 12 selected from the group consisting of
1-Benzyl-5-hydroxy-2-methyl-1H-indole-3-carboxylic Acid, 3,5-Difluorobenzylamide;
1-Furan-2-ylmethyl-5-hydroxy-2-methyl-1H-indole-3-carboxylic Acid 3, 5-Difluorobenzylamide;
5-Hydroxy-2-methyl-1-thiophen-2-ylmethyl-1H-indole-3-carboxylic Acid, 3-Methoxybenzylamide;
1-Benzyl-2-ethyl-5-hydroxy-1H-indole-3-carboxylic Acid, 3,4-Difluoro-benzylamide;
1-Benzyl-2-ethyl-5-hydroxy-1H-indole-3-carboxylic Acid 3,5-Difluoro-benzylamide;
1-Benzyl-5-hydroxy-2-isopropyl-1H-indole-3-carboxylic Acid, 3,5-difluorobenzylamide;
1-Benzyl-5-hydroxy-2-isopropyl-1H-indole-3-carboxylic Acid, 3-methoxybenzylamide; and
1-Benzyl-5-hydroxy-2-phenyl-1H-indole-3-carboxylic Acid, 3,5-Difluorobenzylamide.

14. A compound according to any preceding claim for use in a method of treating a disease or condition selected from the group consisting of glaucoma, dry eye, angiogenesis, cardiovascular conditions and diseases, and wound healing.

## Patentansprüche

1. Verbindungen mit biologischer Sphingosin-1-phosphatrezeptor-Agonist- und/oder Antagonist-Aktivität, dargestellt durch die allgemeine Formel: 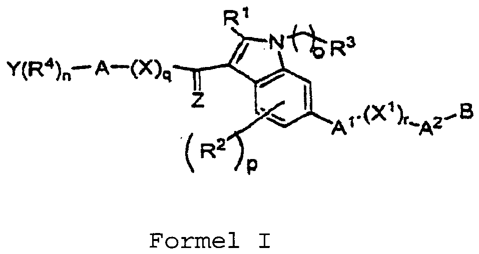 wobei:
R¹, R², R³ und R⁴ unabhängig ausgewählt sind aus der Gruppe, bestehend aus Wasserstoff, gerad- oder verzweigtkettigem Alkyl mit 1 bis 12 Kohlenstoffen, Alkenyl mit 2 bis 6 Kohlenstoffen und 1 oder 2 Doppelbindungen, Alkinyl mit 2 bis 6 Kohlenstoffen und 1 oder 2 Dreifachbindungen, carbocyclischen Kohlenwasserstoffgruppen mit 3 bis 20 Kohlenstoffatomen, heterocyclischen Gruppen mit bis zu 20 Kohlenstoffatomen und mindestens einem Sauerstoff, Stickstoff und/oder Schwefel in dem Ring, Halo-, C₁-C₁₂-Haloalkyl-, Hydroxyl-, C₁-C₁₂-Alkoxy-, C₃-C₂₀-Arylalkyloxy-, C₁-C₁₂-Alkylcarbonyl-, Formyl-, Oxycarbonyl-, Carboxy-, C₁-C₁₂-Alkylcarboxylat-, C₁-C₁₂-Alkylamid-, Aminocarbonyl-, Amino-, Cyano-, Diazo-, Nitro-, Thio-, Sulfoxyl- und Sulfonylgruppen;
X NH ist;
X¹ ausgewählt ist aus der Gruppe bestehend aus NR⁵, O und S;
R⁵ ein Wasserstoff, eine Alkylgruppe mit 1 bis 10 Kohlenstoffen, eine Cycloalkylgruppe mit 5 bis 10 Kohlenstoffen, Phenyl oder Alkylphenyl ist, wobei die Alkylgruppe 1 bis 7 Kohlenstoffatome hat;
Y eine Phenylgruppe oder eine Pyridylgruppe ist, die jeweils an einer beliebigen Position an A gebunden sein kann;
Z O ist;
n 0 oder eine ganze zahl von 1 bis 5 ist;
o 0 oder eine ganze Zahl von 1 bis 3 ist;
p 0 oder eine ganze Zahl von 1 bis 3 ist;
q 1 ist;
r 0 oder 1 ist;
A CH₂ ist;
A¹ und A² unabhängig ausgewählt sind aus der Gruppe, bestehend aus (CH₂)ᵥ,
wobei v 0 oder eine ganze Zahl von 1 bis 12 ist, verzweigtkettigem Alkyl mit 3 bis 12 Kohlenstoffen, Cycloalkyl mit 3 bis 12 Kohlenstoffen, Alkenyl mit 2 bis 10 Kohlenstoffen und 1 bis 3 Doppelbindungen und Alkinyl mit 2 bis 10 Kohlenstoffen und 1 bis 3 Dreifachbindungen;
B ausgewählt ist aus der Gruppe, bestehend aus Wasserstoff, OR⁶, COOR⁷, NR⁸R⁹, CONR⁸R⁹, COR¹⁰, CH=NOR¹¹, CH=NNR¹²R¹³,
wobei R⁶, R⁷, R¹⁰ und R¹¹ unabhängig ausgewählt sind aus der Gruppe bestehend aus Wasserstoff, gerad- oder verzweigtkettigem Alkyl mit 1 bis 12 Kohlenstoffen, Alkenyl mit 2 bis 6 Kohlenstoffen und 1 oder 2 Doppelbindungen, Alkinyl mit 2 bis 6 Kohlenstoffen und 1 oder 2 Dreifachbindungen, einer carbocyclischen Kohlenwasserstoffgruppe mit 3 bis 20 Kohlenstoffatomen, einer heterocyclischen Gruppe mit bis zu 20 Kohlenstoffatomen und mindestens einem Sauerstoff, Stickstoff und/oder Schwefel in dem Ring, R⁸, R⁹, R¹² und R¹³ unabhängig ausgewählt sind aus der Gruppe, bestehend aus Wasserstoff, gerad- oder verzweigtkettigem Alkyl mit 1 bis 12 Kohlenstoffen, Alkenyl mit 2 bis 6 Kohlenstoffen und 1 oder 2 Doppelbindungen, Alkinyl mit 2 bis 6 Kohlenstoffen und 1 oder 2 Dreifachbindungen, einer carbocyclischen Kohlenwasserstoffgruppe mit 3 bis 20 Kohlenstoffatomen, einer heterocyclischen Gruppe mit bis zu 20 Kohlenstoffatomen und mindestens einem Sauerstoff, Stickstoff und/oder Schwefel in dem Ring, oder R⁸ und R⁹ und/oder R¹² und R¹³ zusammen ein zweiwertiges Kohlenstoffradikal mit 2 bis 5 Kohlenstoffen bilden können, um einen heterocyclischen Ring mit Stickstoff zu bilden, wobei jedes von R⁶, R⁷, R⁸, R⁹, R¹⁰, R¹¹, R¹² oder R¹³ mit einem oder mehreren Halogen-, Hydroxy-, Alkyloxy-, Cyano-, Nitro-, Mercapto- oder Thiolradikalen substituiert sein kann; jedoch mit der Maßgabe, dass, wenn v 0 ist und r 0 ist, B kein Wasserstoff ist; oder B eine carbocyclische Kohlenwasserstoffgruppe mit 3 bis 20 Kohlenstoffatomen oder eine heterocyclische Gruppe mit bis zu 20 Kohlenstoffatomen und mindestens einem Sauerstoff, Stickstoff und/oder Schwefel in dem Ring ist, und wobei, wenn B eine carbocyclische oder heterocyclische Gruppe ist, B an einer beliebigen Position an A² gebunden sein kann,
oder ein pharmazeutisch annehmbares Salz der Verbindung.

2. Verbindung gemäß Anspruch 1, wobei n 0 oder eine ganze Zahl von 1 oder 2 ist und R⁴ Fluor ist.

3. Verbindung gemäß Anspruch 2, wobei R¹ i-Propyl ist.

4. Verbindung gemäß Anspruch 3, wobei R³ ausgewählt ist aus der Gruppe, bestehend aus Phenyl, das mit einer oder zwei Fluorgruppen substituiert sein kann, und Pyridyl.

5. Verbindung gemäß Anspruch 4, wobei p 0 ist.

6. Verbindung gemäß Anspruch 5, wobei A¹ und A² nicht vorhanden sind.

7. Verbindung gemäß Anspruch 6, wobei B OR⁶ oder COOR⁷ ist.

8. Verbindung gemäß Anspruch 6, wobei X¹ O ist, r 1 ist, A¹ nicht vorhanden ist, A² (CH₂)ᵥ ist, wobei v 1 oder 2 ist und B OR⁶ oder NR⁸R⁹ ist.

9. Verbindung gemäß Anspruch 8, wobei R⁶, R⁸ und R⁹ Methyl sind.

10. Verbindung gemäß Anspruch 6, wobei B CR¹⁰=NOR¹¹R¹⁰ ist, wobei R¹⁰ H ist und R¹¹ Methyl oder i-Butyl ist, oder wobei B CONR⁸R⁹ ist, wobei R⁸ und R⁹ ausgewählt sind aus der Gruppe, bestehend aus H, Methyl, Ethyl und Propyl, oder R⁸ und R⁹ zusammen mit N einen 5-gliedrigen Ring bilden.

11. Verbindung gemäß Anspruch 6, wobei A¹ nicht vorhanden ist, r 0 ist, A² CH₂ ist und B OR⁶ ist, wobei R⁶ H ist.

12. Verbindung, ausgewählt aus der Gruppe, bestehend aus
1-Benzyl-5-hydroxy-2-methyl-1H-indol-3-carbonsäure-3,5-difluorbenzylamid;
5-Hydroxy-2-methyl-1-thiophen-2-ylmethyl-1H-indol-3-carbonsäure-3,4-difluorbenzylamid;
1-Butyl-5-hydroxy-3-methyl-1H-indol-3-carbonsäure-3,5-difluorbenzylamid;
1-Furan-2-ylmethyl-5-hydroxy-2-methyl-1H-indol-3-carbonsäure-3,4-difluorbenzylamid;
5-Hydroxy-2-methyl-1-thiophen-2-ylmethyl-1H-indol-3-carbonsäure-3,5-difluorbenzylamid;
1-Furan-2-ylmethyl-5-hydroxy-2-methyl-1H-indol-3-carbonsäure-3,5-difluorbenzylamid;
1-Benzyl-5-hydroxy-2-methyl-1H-indol-3-carbonsäure-3,4-difluorbenzylamid
5-Hydroxy-2-methyl-1-thiophen-2-ylmethyl-1H-indol-3-carbonsäure-3-fluorbenzylamid;
5-Hydroxy-2-methyl-1-thiophen-2-ylmethyl-1H-indol-3-carbonsäure-benzylamid
5-Hydroxy-2-methyl-1-thiophen-2-ylmethyl-1H-indol-3-carbonsäure-3-methoxybenzylamid;
1-Butyl-5-hydroxy-2-methyl-1H-indol-3-carbonsäure-3-methoxybenzamid;
5-Hydroxy-2-methyl-1-thiophen-2-ylmethyl-1H-indol-3-carbonsäure-4-fluorbenzylamid;
5-Hydroxy-2-methyl-1-thiophen-2-ylmethyl-1H-indol-3-carbonsäure-4-methylbenzylamid;
5-Hydroxy-2-methyl-1-thiophen-2-ylmethyl-1H-indol-3-carbonsäure-3-chlorbenzylamid;
5-Hydroxy-2-methyl-1-thiophen-2-ylmethyl-1H-indol-3-carbonsäure-4-chlorbenzylamid;
5-Hydroxy-2-methyl-1-thiophen-2-ylmethyl-1H-indol-3-carbonsäure-2-methoxybenzylamid;
1-Benzyl-2-ethyl-5-hydroxy-1H-indol-3-carbonsäure-3,4-difluorbenzylamid;
1-Benzyl-2-ethyl-5-hydroxy-1H-indol-3-carbonsäure-3-methoxybenzylamid;
1-Benzyl-5-hydroxy-2-isopropyl-1H-indol-3-carbonsäure-3,4-difluorbenzamid;
5-Hydroxy-2-methyl-1-phenyl-1H-indol-3-carbonsäure-3,4-difluorbenzylamid;
5-Hydroxy-2-methyl-1-pyridin-2-yl-1H-indol-3-carbonsäure-3,4-difluorbenzylamid;
5-Hydroxy-2-methyl-1-thiophen-2-yl-1H-indol-3-carbonsäure-3,4-difluorbenzylamid;
1-Benzyl-2-ethyl-5-hydroxy-1H-indol-3-carbonsäure-3,5-difluorbenzylamid;
1-Benzyl-5-hydroxy-2-isopropyl-1H-indol-3-carbonsäure-3,5-difluorbenzylamid;
1-Benzyl-5-hydroxy-2-isopropyl-1H-indol-3-carbonsäure-3-methoxybenzylamid; und
1-Benzyl-5-hydroxy-2-phenyl-1H-indol-3-carbonsäure-3,5-difluorbenzylamid.

13. Verbindung gemäß Anspruch 12, ausgewählt aus der Gruppe, bestehend aus
1-Benzyl-5-hydroxy-2-methyl-1H-indol-3-carbonsäure-3,5-difluorbenzylamid;
1-Furan-2-ylmethyl-5-hydroxy-2-methyl-1H-indol-3-carbonsäure-3,5-difluorbenzylamid;
5-Hydroxy-2-methyl-1-thiophen-2-ylmethyl-1H-indol-3-carbonsäure-3-methylbenzylamid;
1-Benzyl-2-ethyl-5-hydroxy-1H-indol-3-carbonsäure-3,4-difluorbenzylamid;
1-Benzyl-2-ethyl-5-hydroxy-1H-indol-3-carbonsäure-3,5-difluorbenzylamid;
1-Benzyl-5-hydroxy-2-isopropyl-1H-indol-3-carbonsäure-3,5-difluorbenzylamid;
1-Benzyl-5-hydroxy-2-isopropyl-1H-indol-3-carbonsäure-3-methoxybenzylamid; und
1-Benzyl-5-hydroxy-2-isopropyl-1H-indol-3-carbonsäure-3,5-difluorbenzylamid.

14. Verbindung gemäß einem der vorangehenden Ansprüche zur Verwendung in einem Verfahren zur Behandlung einer Krankheit oder eines Zustands, ausgewählt aus der Gruppe, bestehend aus Glaukom, trockenem Auge, Angiogenese, Herz-Kreislauf-Zuständen und -Erkrankungen und Wundheilung.

## Revendications

1. Composé, représenté en ce qu'il a une activité biologique d'agoniste et/ou d'antagoniste de récepteur de sphingosine-1-phosphate, représenté par la formule générale : 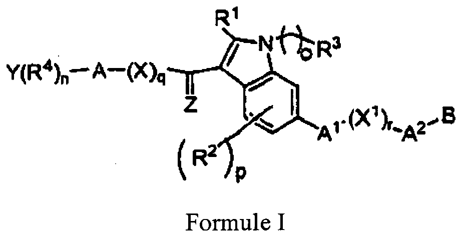 dans lequel
R¹, R², R³ et R⁴ sont indépendamment choisis dans le groupe consistant en un hydrogène, un alkyle à chaîne droite ou ramifiée comportant 1 à 12 atomes de carbone, un alcényle comportant 2 à 6 atomes de carbone, et 1 ou 2 doubles liaisons, un alcynyle comportant 2 à 6 atomes de carbone et 1 ou 2 triples liaisons, des groupes hydrocarbure carbocycliques comportant de 3 à 20 atomes de carbone, des groupes hétérocycliques comportant jusqu'à 20 atomes de carbone et au moins l'un parmi l'oxygène, l'azote et/ou le soufre dans le cycle, les groupes halogéno, halogénoalkyle en C₁ à C₁₂, hydroxyle, alcoxy en C₁ à C₁₂, arylalkyloxy en C₃ à C₂₀, alkylcarbonyle en C₁ à C₁₂, formyle, oxycarbonyle, carboxy, carboxylate d'alkyle en C₁ à C₁₂, alkylamide en C₁ à C₁₂, aminocarbonyle, amino, cyano, diazo, nitro, thio, sulfoxyle et sulfonyle ;
X est NH ;
X¹ est choisi dans le groupe consistant en NR⁵, O et S ;
R⁵ est un hydrogène, un groupe alkyle de 1 à 10 atomes de carbone, un groupe cycloalkyle de 5 à 10 atomes de carbone, phényle ou alkylphényle dans lequel le groupe alkyle comporte 1 à 7 atomes de carbone ;
Y est un groupe phényle ou un groupe pyridyle, dont chacun peut être lié à A en toute position ;
Z est O ;
n vaut 0 ou un entier de 1 à 5 ;
o vaut 0 ou un entier de 1 à 3 ;
p vaut 0 ou un entier de 1 à 3 ;
q vaut 1 ;
r vaut 0 ou 1 ;
A est CH₂ ;
A¹ et A² sont indépendamment choisis dans le groupe consistant en (CH₂)ᵥ où v vaut 0 ou un entier de 1 à 12, un alkyle à chaîne ramifiée comportant 3 à 12 atomes de carbone, un cycloalkyle comportant 3 à 12 atomes de carbone, un alcényle comportant 2 à 10 atomes de carbone et 1 à 3 doubles liaisons et un alcynyle comportant 2 à 10 atomes de carbone et 1 à 3 triples liaisons ;
B est choisi dans le groupe consistant en l'hydrogène, OR⁶, COOR⁷, NR⁸R⁹, CONR⁸R⁹, COR¹⁰, CH=NOR¹¹, CH=NNR¹²R¹³
où R⁶, R⁷, R¹⁰ et R¹¹ sont indépendamment choisis dans le groupe consistant en l'hydrogène, un alkyle à chaîne droite ou ramifiée comportant 1 à 12 atomes de carbone, un alcényle comportant 2 à 6 atomes de carbone et 1 ou 2 doubles liaisons, un alcynyle comportant 2 à 6 atomes de carbone et 1 ou 2 triples liaisons, un groupe hydrocarbure carbocyclique comportant 3 à 20 atomes de carbone, un groupe hétérocyclique comportant jusqu'à 20 atomes de carbone et au moins l'un parmi l'oxygène, l'azote et/ou le soufre dans le cycle, R⁸, R⁹, R¹² et R¹³ sont indépendamment choisis dans le groupe consistant en l'hydrogène, un alkyle à chaîne droite ou ramifiée comportant 1 à 12 atomes de carbone, un alcényle comportant 2 à 6 atomes de carbone et 1 ou 2 doubles liaisons, un alcynyle comportant 2 à 6 atomes de carbone et 1 ou 2 triples liaisons, un groupe hydrocarbure carbocyclique comportant de 3 à 20 atomes de carbone, un groupe hétérocyclique comportant jusqu'à 20 atomes de carbone et au moins l'un parmi l'oxygène, l'azote et/ou le soufre dans le cycle, ou R⁸ et R⁹ et/ou R¹² et R¹³, ensemble, peuvent former un radical carboné divalent de 2 à 5 atomes de carbone, pour former un cycle hétérocyclique avec l'azote, où l'un quelconque de R⁶, R⁷, R⁸, R⁹, R¹⁰, R¹¹, R¹² ou R¹³ peut être substitué avec un ou plusieurs radicaux halogène, hydroxy, alkyloxy, cyano, nitro, mercapto ou thiol ; à condition toutefois que, lorsque v vaut 0, et r vaut 0, B ne soit pas un hydrogène ; ou B soit un groupe hydrocarbure carbocyclique comportant de 3 à 20 atomes de carbone, ou un groupe hétérocyclique comportant jusqu'à 20 atomes de carbone et au moins l'un parmi l'oxygène, l'azote et/ou le soufre dans le cycle, et où lorsque ledit B est un groupe carboxyclique ou hétérocyclique, B soit lié à A² en toute position, ou sel pharmaceutiquement acceptable dudit composé.

2. Composé selon la revendication 1, dans lequel n vaut 0 ou un entier de 1 ou 2 et R⁴ est un fluoro.

3. Composé selon la revendication 2, dans lequel R¹ est un i-propyle.

4. Composé selon la revendication 3, dans lequel R³ est choisi dans le groupe consistant en un phényle, qui peut être substitué par un ou deux groupes fluoro, et un pyridyle.

5. Composé selon la revendication 4, dans lequel p vaut 0.

6. Composé selon la revendication 5, dans lequel A¹ et A² sont absents.

7. Composé selon la revendication 6, dans lequel B est OR⁶ ou COOR⁷.

8. Composé selon la revendication 6, dans lequel X¹ est O, r vaut 1, A¹ est absent, A² est (CH₂)ᵥ, où v vaut 1 ou 2, et B est OR⁶ ou NR⁸R⁹.

9. Composé selon la revendication 8, dans lequel R⁶, R⁸ et R⁹ sont des méthyles.

10. Composé selon la revendication 6, dans lequel B est CR¹⁰=NOR¹¹R¹⁰ où R¹⁰ est H et R¹¹ est un méthyle ou i-butyle, ou dans lequel B est CONR⁸R⁹ où R⁸ et R⁹ sont choisis dans le groupe consistant en H, méthyle, éthyle et propyle, ou R⁸ et R⁹, conjointement avec N, forment un cycle à 5 chaînons.

11. Composé selon la revendication 6, dans lequel A¹ est absent, r vaut 0, A² est CH₂ et B est OR⁶, où R⁶ est H.

12. Composé choisi dans le groupe consistant en
l'acide 1-benzyl-5-hydroxy-2-méthyl-1H-indole-3-carboxylique, 3,5-difluorobenzylamide ;
l'acide 5-hydroxy-2-méthyl-1-thiophén-2-ylméthyl-1H-indole-3-carboxylique, 3,4-difluorobenzylamide ;
l'acide 1-butyl-5-hydroxy-2-méthyl-1H-indole-3-carboxylique, 3,5-difluorobenzylamide ;
l'acide 1-furan-2-ylméthyl-5-hydroxy-2-méthyl-1H-indo 1 e-3-carboxylique, 3,4-difluorobenzylamide ;
l'acide 5-hydroxy-2-méthyl-1-thiophén-2-ylméthyl-1H-indole-3-carboxylique, 3,5-difluorobenzylamide ;
l'acide 1-furan-2-ylméthyl-5-hydroxy-2-méthyl-1H-indole-3-carboxylique, 3,5-difluorobenzylamide ;
l'acide 1-benzyl-5-hydroxy-2-méthyl-1H-indole-3-carboxylique, 3,4-difluorobenzylamide ;
l'acide 5-hydroxy-2-méthyl-1-thiophén-2-ylméthyl-1H-indole-3-carboxylique, 3-fluorobenzylamide ;
l'acide 5-hydroxy-2-méthyl-1-thiophén-2-ylméthyl-1H-indole-3-carboxylique, benzylamide ;
l'acide 5-hydroxy-2-méthyl-1-thiophén-2-ylméthyl-1H-indole-3-carboxylique, 3-méthoxybenzylamide ;
l'acide 1-butyl-5-hydroxy-2-méthyl-1H-indole-3-carboxylique, 3-méthoxybenzylamide ;
l'acide 5-hydroxy-2-méthyl-1-thiophén-2-ylméthyl-1H-indole-3-carboxylique, 4-fluorobenzylamide ;
l'acide 5-hydroxy-2-méthyl-1-thiophén-2-ylméthyl-1H-indole-3-carboxylique, 4-méthylbenzylamide ;
l'acide 5-hydroxy-2-méthyl-1-thiophén-2-ylméthyl-1H-indole-3-carboxylique, 3-chlorobenzylamide ;
l'acide 5-hydroxy-2-méthyl-1-thiophén-2-ylméthyl-1H-indole-3-carboxylique, 4-chlorobenzylamide ;
l'acide 5-hydroxy-2-méthyl-1-thiophén-2-ylméthyl-1H-indole-3-carboxylique, 2-méthoxybenzylamide ;
l'acide 1-benzyl-2-éthyl-5-hydroxy-1H-indole-3-carboxylique, 3,4-difluorobenzylamide ;
l'acide 1-benzyl-2-éthyl-5-hydroxy-1H-indole-3-carboxylique, 3-méthoxybenzylamide ;
l'acide 1-benzyl-5-hydroxy-2-isopropyl-1H-indole-3-carboxylique, 3,4-difluorobenzamide ;
l'acide 5-hydroxy-2-méthyl-1-phenyl-1H-indole-3-carboxylique, 3,4-difluorobenzylamide ;
l'acide 5-hydroxy-2-méthyl-1-pyridin-2-yl-1H-indole-3-carboxylique, 3,4-difluorobenzylamide ;
l'acide 5-hydroxy-2-méthyl-1-thiophén-2-yl-1H-indole-3-carboxylique, 3,4-difluorobenzylamide ;
l'acide 1-benzyl-2-éthyl-5-hydroxy-1H-indole-3-carboxylique, 3,5-difluorobenzylamide ;
l'acide 1-benzyl-5-hydroxy-2-isopropyl-1H-indole-3-carboxylique, 3,5-difluorobenzylamide ;
l'acide 1-benzyl-5-hydroxy-2-isopropyl-1H-indole-3-carboxylique, 3-méthoxybenzylamide ; et
l'acide 1-benzyl-5-hydroxy-2-phényl-1H-indole-3-carboxylique, 3,5-difluorobenzylamide.

13. Composé selon la revendication 12, choisi dans le groupe consistant en
l'acide 1-benzyl-5-hydroxy-2-méthyl-1H-indole-3-carboxylique, 3,5-difluorobenzylamide ;
l'acide 1-furan-2-ylméthyl-5-hydroxy-2-méthyl-1H-indole-3-carboxylique, 3,5-difluorobenzylamide ;
l'acide 5-hydroxy-2-méthyl-1-thiophén-2-ylméthyl-1H-indole-3-carboxylique, 3-méthoxybenzylamide ;
l'acide 1-benzyl-2-éthyl-5-hydroxy-1H-indole-3-carboxylique, 3,4-difluorobenzylamide ;
l'acide 1-benzyl-2-éthyl-5-hydroxy-1H-indole-3-carboxylique, 3,5-difluorobenzylamide ;
l'acide 1-benzyl-5-hydroxy-2-isopropyl-1H-indole-3-carboxylique, 3,5-difluorobenzylamide ;
l'acide 1-benzyl-5-hydroxy-2-isopropyl-1H-indole-3-carboxylique, 3-méthoxybenzylamide ; et
l'acide 1-benzyl-5-hydroxy-2-phenyl-1H-indole-3-carboxylique, 3,5-difluorobenzylamide.

14. Composé selon l'une quelconque des revendications précédentes, à utiliser dans une méthode de traitement d'une maladie ou affection choisie dans le groupe constitué de : le glaucome, le syndrome de l'oeil sec, l'angiogenèse, les affections et maladies cardiovasculaires et la cicatrisation de plaies.
